# DEMANDE DE BREVET EUROPEEN

(11) **EP 1 493 739 A1**
(43) Date de publication de la demande: **05.01.2005**
(21) Numéro de dépôt: 03291641.3
(22) Date de dépôt: 03.07.2003
(51) Int. Cl.: C07D 333/38, C07D 409/10, A61K 31/381, A61K 31/4436, A61P 11/00

(54) **Dérivés thiophényliques d'aminoacides, leur procédé de préparation et les compositions pharmaceutiques qui les contiennent**

(71) Demandeur: Warner-Lambert Company LLC, Morris Plains, New Jersey 07950 (US)
(72) Inventeur: Compère, Delphine, c/o Pfizer PGRD Patent Dept., 94265 Fresnes Cédex (FR); Dublanchet, A., c/o Pfizer PGRD Patent Dept., 94265 Fresnes Cédex (FR); Ducrot, Pierre, c/o Pfizer PGRD Patent Dept., 94265 Fresnes Cédex (FR); Cluzeau, Philippe, c/o Pfizer PGRD Patent Dept., 94265 Fresnes Cédex (FR); Courté Karine, c/o Pfizer PGRD Patent Dept., 94265 Fresnes Cédex (FR); Blais, Stéphane, c/o Pfizer PGRD Patent Dept., 94265 Fresnes Cédex (FR); Denis, Alexis, c/o Pfizer PGRD - Patent Department, 94265 Fresnes Cedex (FR)
(74) Mandataire: Dekker, Henrike

(57) **Abrégé**

Composés de formule (I) : 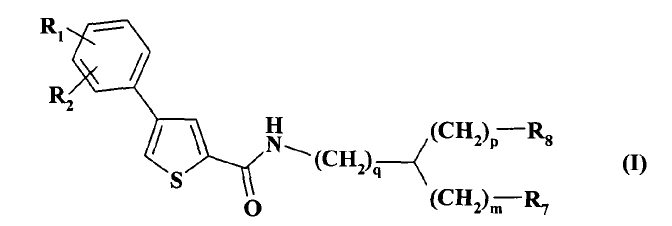 dans laquelle:
- R₁ et R₂, identiques ou différents, indépendamment l'un de l'autre, représentent chacun un groupement choisi parmi :
   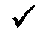 hydrogène, halogène, cyano, nitro, halogéno(C₁-C₆)alkyle, halogéno(C₁-C₆)alkoxy, (C₁-C₆)alkyle, (C₂-C₆)alkènyle, (C₂-C₆)alkynyle,
   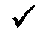 -OR₄, -NR₄R₅, -S(O)ₙR₄, -C(O)R₄, -CO₂R₄, -O-C(O)R₄, -C(O)NR₄R₅, -NR₅-C(O)R₄, -NR₅-SO₂R₄, -T-CN, -T-OR₄, -T-OCF₃, -T-NR₄R₅, -T-S(O)ₙR₄, -T-C(O)R₄, -T-CO₂R₄, -T-O-C(O)R₄, -T-C(O)NR₄R₅, -T-NR₅-C(O)R₄, -T-NR₅-SO₂R₄, -R₆, et -T-R₆ dans lesquels n, T, R₄, R₅ et R₆ sont tels que définis dans la description ;
- m représente un entier compris entre 0 et 4 inclus ;
- p représente un entier compris entre 0 et 4 inclus ;
- q représente un entier compris entre 0 et 4 inclus ;
- R₇ représente un groupement choisi parmi aryle, cycloalkyle et un hétérocycle chacun de ces groupements étant éventuellement substitué;
- R₈ représente un groupement choisi parmi carboxy, (C₁-C₆)alkoxycarbonyl, (C₁-C₇)acyle, hydroxy(C₁-C₆)alkylcarbonyl, (C₁-C₆)alkoxy(C₁-C₆)alkylcarbonyl, tetrazole, amino, aminocarbonyl, et amino(C₁-C₆)alkylcarbonyl;
leurs isomères, ainsi que leurs sels d'addition à un acide ou à une base pharmaceutiquement acceptable, et les compositions pharmaceutiques utiles pour le traitement des pathologies liées à une inhibition des métalloprotéinases.

## Description

La présente invention concerne de nouveaux dérivés d'aminoacide thiophènes, leur procédé de préparation et les compositions pharmaceutiques qui les contiennent.

Les composés de la présente invention sont particulièrement intéressants d'un point de vue pharmacologique pour leur interaction spécifique avec les métalloprotéinases et plus spécifiquement avec la métalloélastase macrophagique (MMP-12), trouvant leur application dans la prévention et le traitement des pathologies respiratoires telles que les broncho-pneumopathies chroniques obstructives (BPCO), l'emphysème, les bronchites chroniques, les inflammations pulmonaires chroniques, l'asthme, la fibrose cystique, la détresse respiratoire aiguë (ARDS), les allergies respiratoires dont la rhinite allergique, ainsi que les maladies liées à la production de TNFα incluant les maladies pulmonaires fibrotiques sévères, la sarcoïdose pulmonaire, et la silicose.

Les composés de la présente invention montrent aussi, à un niveau moindre, une activité inhibitrice de la métalloprotéinase-13 (MMP-13) les rendant potentiellement utiles pour le traitement des pathologies impliquant cette enzyme, telles que le cancer, l'ostéoporose, l'ostéoarthrite, l'arthrite, l'arthrite rhumatoïde, l'athéorosclérose, la sclérose en plaques, l'insuffisance cardiaque.

Les métalloprotéinases (MMPs) constituent une large famille de proteinases dégradant la matrice extracellulaire et sont sécrétées notamment par les cellules mésenchymales, les macrophages et les leucocytes polynucléaires. Les métalloprotéinases sont classées en plusieurs sous-familles selon leur structure primaire et leur spécificité. Ces familles incluent notamment les collagénases (MMP-1, MMP-8 et MMP-13), les stromélysines (MMP-3 et MMP-10), les gélatinases (MMP-2 et MMP-9), la matrilysine (MMP-7), la métalloélastase macrophagique (MMP-12) ainsi que les MMPs de type membranaire (MMP-14, MMP-15, MMP-16 et MMP-17).

Les MMPs sont des zinc-métalloprotéinases ayant la capacité de dégrader la quasi-totalité des composants de la matrice extracellulaire, c'est-à-dire l'interstitium et les membranes basales. Une synthèse accrue de ces enzymes est retrouvée dans de nombreuses maladies destructrices (arthrite inflammatoire, athérosclérose, invasion tumorale, angiogénèse). Les MMPs (en particulier celles ayant une puissante activité élastolytique) sont impliquées dans la physiopathologie de l'asthme et des broncho-pneumopathies chroniques obstructives dont l'emphysème pulmonaire lié au tabac (BPCO).

L'élastase macrophagique humaine (HME ou MMP-12) présente toutes les caractéristiques des autres MMPs. Elle dégrade de nombreuses macromolécules de la matrice extracellulaire (gélatine, fibronectine et laminine) et surtout l'élastine. La MMP-12 n'est pas synthétisée par les cellules monocytaires circulantes, mais uniquement par les macrophages ou encore les monocytes différenciés *in vitro* en macrophages. La pathologie de l'emphysème est caractérisée par la destruction de l'élastine présente dans les parois des alvéoles pulmonaires. La mise en évidence de l'augmentation du taux de MMP-12 lors de la manifestation de cette pathologie, suggère ainsi un rôle prédominant de cette enzyme dans la survenue et le développement de cette maladie. De même des études ont démontré l'absence de développement d'emphysème chez des souris déficientes en MMP-12, ces souris étant exposées longuement à la fumée de cigarette (*Science* **1997**, 277, 2002-2004). Plus récemment, en utilisant également des souris déficientes en MMP-12, dans un modèle d'asthme, un groupe a suggéré l'implication de la MMP-12 dans le développement de l'asthme chronique (*FASEB,* **2002**, 16, A590). Ces résultats démontrent clairement que des inhibiteurs de l'élastase macrophagique humaine (MMP-12) pourraient être très utiles pour la prévention et le traitement de pathologies respiratoires chroniques telles que la bronchite pulmonaire chronique obstructive (BPCO), l'emphysème, les bronchites chroniques, les inflammations pulmonaires chroniques, mais également les pathologies respiratoires dues à un phénomène d'inflammation telles que l'asthme, la mucoviscidose, la détresse respiratoire aiguë (ARDS), les allergies respiratoires dont la rhinite allergique, ainsi que les maladies liées à la production de TNFα incluant les maladies pulmonaires fibrotiques sévères, la sarcoïdose pulmonaire, et la silicose.

Toutes les métalloprotéinases présentent un domaine catalytique constitué de 162 à 173 acides aminés contenant le site actif de l'enzyme. Un ion Zn²⁺ est présent dans le site actif auquel il est fixé par l'intermédiaire de résidus histidines. Ce site constitue l'un des points d'ancrage privilégié des inhibiteurs synthétiques des MMPs car il permet notamment de créer un centre de chélation stable, puissant et aisément accessible pour les petites molécules. Ainsi tous les inhibiteurs puissants décrits dans la littérature possèdent une fonction chimique telle qu'un acide hydroxamique permettant une chélation entre l'atome de zinc du site catalytique de la métalloprotéinase et ledit inhibiteur. Cette chélation assure un blocage du site actif et entraîne l'inhibition de ladite enzyme.

L'un des problèmes majeurs de ce type d'inhibition est l'absence de sélectivité ou le faible taux de sélectivité, toutes les MMPs possédant en effet un ion zinc au sein de leur site actif. Le second problème lié à ces inhibiteurs puissants mais généralement faiblement sélectifs est la toxicité liée à la présence d'une fonction chimique telle qu'un acide hydroxamique.

L'un des objets de l'invention est donc de fournir de nouveaux composés possédant des propriétés inhibitrices de la métalloprotéinase de type 12 (MMP-12). Une solution a été trouvée par l'élaboration de nouveaux dérivés d'aminoacide thiophènes, ainsi que par l'utilisation desdits composés dans des compositions pharmaceutiques aptes à être utilisées dans la prévention et le traitement des pathologies liées à une inhibition de la MMP-12.

Plusieurs demandes de brevets ou articles scientifiques décrivent des composés comportant un motif central thiophène. Parmi cette littérature il peut être cité la demande de brevet WO 98/23605 qui décrit des dérivés thién-2-yl-carboxamides substitués en position 4 par un système cyclique et en position 5 par un groupement trifluorométhyle. Ces composés sont revendiqués pour leur activité bactéricide et fongicide. La demande de brevet WO 96/16954 décrit également des composés comportant éventuellement un système 4-aryl-thién-2-yl carboxamide dans lequel la fonction amide peut être substituée par un groupement phényle, utiles pour leur propriété anti-fongique.

Aucun de ces documents ne décrit ou ne suggère pour ces composés une activité inhibitrice de la MMP-12 ou une activité inhibitrice mixte MMP-12/MMP-13 et une utilisation potentielle de ce type de produit dans le traitement de pathologies respiratoires, ou de pathologies de type inflammatoire, propriété originale des composés revendiqués par la demanderesse.

Plus particulièrement, la présente invention concerne les composés de formule (I) : 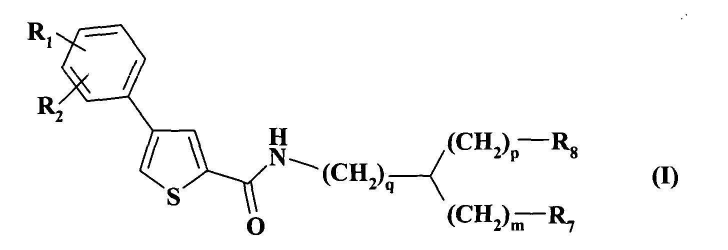 dans laquelle:
- R₁ et R₂, identiques ou différents, indépendamment l'un de l'autre, représentent chacun un groupement choisi parmi :
   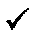 hydrogène, halogène, cyano, nitro, halogéno(C₁-C₆)alkyle, halogéno(C₁-C₆)alkoxy, (C₁-C₆)alkyle, (C₂-C₆)alkènyle, (C₂-C₆)alkynyle,
   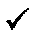 -OR₄, -NR₄R₅, -S(O)ₙR₄, -C(O)R₄, -CO₂R₄, -O-C(O)R₄, -C(O)NR₄R₅, -NR₅-C(O)R₄, - NR₅-SO₂R₄, -T-CN, -T-OR₄, -T-OCF₃, -T-NR₄R₅, -T-S(O)ₙR₄, -T-C(O)R₄, - T-CO₂R₄, -T-O-C(O)R₄, -T-C(O)NR₄R₅, -T-NR₅-C(O)R₄, -T-NR₅-SO₂R₄, -R₆, et - T-R₆ dans lesquels :
      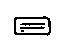 n représente un entier compris entre 0 et 2 inclus,
      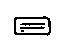 T représente une chaîne (C₁-C₆)alkylène linéaire ou ramifiée, éventuellement substituée par un groupement choisi parmi oxo, halogène, (C₁-C₆)alkoxy, hydroxy, amino, mono(C₁-C₆)alkylamino et di(C₁-C₆)alkylamino, et/ou dans laquelle éventuellement un des atomes de carbone est remplacé par un atome d'oxygène, un atome de soufre, un groupement -NH-, ou un groupement - N(C₁-C₆)alkyle-, (étant entendu que dans le cas où un des atomes de carbone est remplacé par un groupement tel que défini précédemment alors ladite chaîne alkylène comporte au moins un enchaînement de deux atomes)
      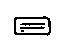 R₄ représente un atome d'hydrogène, un groupement (C₁-C₆)alkyle, aryle, cycloalkyle, ou un hétérocycle,
      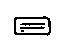 R₅ représente un atome d'hydrogène ou un groupement (C₁-C₆)alkyle,
      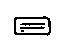 R₆ représente un groupement choisi parmi aryle, cycloalkyle, et un hétérocyle, chacun de ces groupements étant éventuellement substitués par un à cinq groupements, identiques ou différents, indépendamment l'un de l'autre choisis parmi halogène, cyano, nitro, oxo, halogéno(C₁-C₆)alkyle, halogéno(C₁-C₆)alkoxy, (C₁-C₆)alkyle, (C₁-C₆)alkènyle, -OR₄₀, -NR₄₀R₅₀, -S(O)ₙ₁R₄₀, - C(O)R₄₀, -CO₂R₄₀, -O-C(O)R₄₀, -C(O)NR₄₀R₅₀, -NR₅₀-C(O)R₄₀, -NR₅₀-SO₂R₄₀, -T₁-CN, -T₁-OR₄₀, -T₁-OCF₃, -T₁-NR₄₀R₅₀, -T₁-S(O)ₙR₄₀, - T₁-C(O)R₄₀, -T₁-CO₂R₄₀, -T₁-O-C(O)R₄₀, -T₁-C(O)NR₄₀R₅₀,
      - T₁-NR₅₀-C(O)R₄₀, et -T₁-NR₅₀-SO₂R₄₀ dans lesquels R₄₀, R₅₀, T₁ et n₁ ont respectivement les mêmes significations que R₄, R₅, T et n tels que définis précédemment ;
- m représente un entier compris entre 0 et 4 inclus ;
- p représente un entier compris entre 0 et 4 inclus ;
- q représente un entier compris entre 0 et 4 inclus ;
- R₇ représente un groupement choisi parmi aryle, cycloalkyle et un hétérocycle chacun de ces groupements étant éventuellement substitué par un à trois groupements, identiques ou différents, indépendamment l'un de l'autre choisis parmi parmi halogène, cyano, nitro, halogéno(C₁-C₆)alkyle, halogéno(C₁-C₆)alkoxy, (C₁-C₆)alkyle, hydroxy, (C₁-C₆)alkoxy, amino, mono(C₁-C₆)alkylamino, di(C₁-C₆)alkylamino, mercapto, (C₁-C₆)alkylthio, (C₁-C₇)acyle, (C₁-C₆)alkylsulfoxyde, carboxy, et (C₁-C₆)alkoxycarbonyl ;
- R₈ représente un groupement choisi parmi carboxy, (C₁-C₆)alkoxycarbonyl, (C₁-C₇)acyle, hydroxy(C₁-C₆)alkylcarbonyl, (C₁-C₆)alkoxy(C₁-C₆)alkylcarbonyl, tetrazole, amino, aminocarbonyl, et amino(C₁-C₆)alkylcarbonyl, (la partie amino dans chacun des groupements portant cette fonction étant éventuellement substituée par un ou deux groupements, identiques ou différents, indépendamment l'un de l'autre choisis parmi (C₁-C₆)alkyl et cycloalkyle) ;
leurs isomères, ainsi que leurs sels d'addition à un acide ou à une base pharmaceutiquement acceptable, étant entendu que dans la définition générale des différents groupements des composés de formule (I):
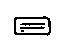 par groupement aryle on comprend un système monocyclique ou bicyclique, aromatique comprenant de 4 à 10 atomes de carbone, étant compris que dans le cas d'un système bicyclique l'un des cycles présente un caractère aromatique et l'autre cycle est aromatique ou insaturé ; à titre indicatif il peut être cité les groupements suivants : phényle, naphthyle, indényle, benzocyclobutènyle, 1,2,3,4-tétrahydronaphtyl, ...
   - par groupement cycloalkyle on comprend un système monocyclique ou bicyclique, fusionné ou ponté, saturé ou partiellement insaturé, comprenant de 3 à 12 atomes de carbone ; à titre indicatif il peut être cité les groupements suivants : cyclopropyle, cyclobutyle, cyclopentyle, cyclohexyle, cycloheptyle, cyclooctyle, adamantyle, décalinyle, norbomyle, cyclopentènyle, cyclohexènyl, cyclohexènediyl, ...
   - par un hétérocycle on comprend un système monocyclique ou bicyclique, fusionné ou ponté, de 3 à 12 chaînons, saturé, insaturé ou aromatique, comprenant de 1 à 4 hétéroatomes, identiques ou différents, indépendamment l'un de l'autre choisis parmi oxygène, soufre et azote, et contenant éventuellement 1 ou 2 groupements oxo ou thioxo, étant compris que dans le cas d'un système bicyclique l'un des cycles peut présenter un caractère aromatique et l'autre cycle est aromatique ou insaturé, ou les deux cycles sont saturés, ou l'un des cyles est saturé et l'autre cycle est insaturé, ou les deux cycles sont insaturés ; à titre indicatif il peut être cité les groupements suivants : furyle, thiènyle, pyrrolyle, pyrazolyle, pyridyle, pyrimidyle, pyrazinyle, benzofuryle, benzothiènyle, indolyle, quinolyle, isoquinolyle, imidazolyle, benzodioxolyle, benzodioxinyle, benzo[1,2,5]thiadiazolyle, benzo[1,2,5]oxadiazolyle, [1,2,3]triazolyle, [1,2,4]triazolyle, morpholinyl, piperidyle, piperazinyle, pyrrolidinyle, ...
   - par groupement (C₁-C₆)alkyle on comprend une chaîne carbonée linéaire ou ramifiée comprenant de 1 à 6 atomes de carbone; à titre indicatif il peut être cité les groupements suivants : méthyle, éthyle, propyle, isopropyle, tert-butyle, néopentyle, hexyle,...
   - par groupement (C₂-C₆)alkènyle on comprend une chaîne carbonée linéaire ou ramifiée comprenant de 2 à 6 atomes de carbone et une ou plusieurs double liaisons ; à titre indicatif il peut être cité les groupements suivants : vinyle, allyle, 3-butèn-1-yl, 2-méthyl-butèn-1-yl, hexènyl,...
   - par groupement (C₂-C₆)alkynyl on comprend une chaîne carbonée linéaire ou ramifiée comprenant de 2 à 6 atomes de carbone et une ou plusieurs triples liaisons ; à titre indicatif il peut être cité les groupements suivants : éthynyle, propynyle, 3-butyn-1-yl, 2-méthyl-butyn-1-yl, hexynyl,...
   - par groupement (C₁-C₆)alkoxy on comprend un groupement alkyle tel que défini précédemment lié au travers d'un atome d'oxygène ; à titre indicatif il peut être cité les groupements suivants : méthoxy, éthoxy, *n*-propyloxy, *tert*-butyloxy,...
   - par groupement halogéno(C₁-C₆)alkyle on comprend une chaîne carbonée linéaire ou ramifiée comprenant de 1 à 6 atomes de carbone et substituée par 1 à 6 atomes d'halogène; à titre indicatif il peut être cité les groupements suivants : trifluorométhyle, 2,2,2-trifluoroéthyle, ...
   - par groupement halogéno(C₁-C₆)alkoxy on comprend une chaîne carbonée linéaire ou ramifiée comprenant de 1 à 6 atomes de carbone et substituée par 1 à 6 atomes d'halogène, ladite chaîne étant reliée au composé de formule (I) par un atome d'oxygène ; à titre indicatif il peut être cité les groupements suivants : trifluorométhoxy, 2,2,2-trifluoroéthoxy, ...
   - par atome d'halogène on comprend un atome choisi parmi brome, chlore, fluor et iode,
   - par groupement acyle on comprend un atome d'hydrogène, un groupement alkyle tel que défini précédemment, un cycloalkyle de 3 à 6 atomes de carbone, ou un groupement phényle lié au travers d'un groupement oxo aux composés de formule (I); à titre indicatif il peut être cité les groupements suivants : formyle, acétyle, éthylcarbonyle, n-propylcarbonyle, *tert*-butylcarbonyle, cyclopropylcarbonyle, benzoyle, ...
   - par système cyclique on comprend les groupements aryles, cycloalkyles et les hétérocycles tels que définis précédemment,
   - les isomères optiques se réfèrent aux racémates, énantiomères et diastéréoisomères.

Selon une variante avantageuse de l'invention, les composés préférés de l'invention sont les composés de formule (I) dans laquelle :
- R₁ représente un groupement choisi parmi halogéno(C₁-C₆)alkoxy, (C₁-C₆)alkyle, - OR₄, -SR₄, et -R₆ dans lesquel :
   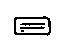 R₄ représente un atome d'hydrogène ou un groupement (C₁-C₆)alkyl,
   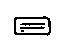 R₆ représente un groupement choisi parmi phényle, cyclohexyle, et un hétérocycle, chacun de ces groupements étant éventuellement substitués par un à deux groupements, identiques ou différents, indépendamment l'un de l'autre choisis parmi halogène, halogéno(C₁-C₆)alkyl-, halogéno(C₁-C₆)alkoxy-, (C₁-C₆)alkyle, cyano, -T₁-CN, -OR₄₀, -T₁-OR₄₀, -NR₄₀R₅₀, -S(O)ₙ₁R₄₀, et - C(O)R₄₀, dans lesquels :
      - R₄₀ représente un groupement choisi parmi atome d'hydrogène, (C₁-C₆)alkyl et phényle,
      - Rso représente un groupement choisi parmi atome d'hydrogène et (C₁-C₆)alkyl,
      - T₁ représente une chaîne (C₁-C₆)alkylène linéaire ou ramifiée,
      - n1 représente un entier compris entre 0 et 2 inclus ;
- R₂ représente un atome d'hydrogène ;
- m représente un entier compris entre 0 et 4 inclus ;
- p représente un entier compris entre 0 et 4 inclus ;
- q représente un entier compris entre 0 et 4 inclus ;
- R₇ représente un groupement choisi parmi phényle, cycloalkyle monocyclique, et un hétérocycle monocyclique à 5 ou 6 chaînons et comprenant un ou deux hétéroatomes, identiques ou différents, indépendamment l'un de l'autre, choisis parmi oxygène et azote, chacun de ces groupements étant éventuellement substitué par un à deux groupements, identiques ou différents, indépendamment l'un de l'autre, choisis parmi parmi halogène, cyano, nitro, halogéno(C₁-C₆)alkyle, halogéno(C₁-C₆)alkoxy, (C₁-C₆)alkyle, hydroxy, (C₁-C₆)alkoxy, amino, mono(C₁-C₆)alkylamino, di(C₁-C₆)alkylamino, mercapto, (C₁-C₆)alkylthio, (C₁-C₇)acyle, (C₁-C₆)alkylsulfoxyde, carboxy, et (C₁-C₆)alkoxycarbonyl ;
- R₈ représente un groupement choisi parmi carboxy, (C₁-C₆)alkoxycarbonyl, aminocarbonyl, (C₁-C₆)alkylcarbonyl, amino, hydroxy(C₁-C₆)alkylcarbonyl, (C₁-C₆)alkoxy(C₁-C₆)alkylcarbonyl, amino(C₁-C₆)alkylcarbonyl, (la partie amino dans chacun des groupements portant cette fonction étant éventuellement substituée par un ou deux groupements, identiques ou différents, indépendamment l'un de l'autre (C₁-C₆)alkyl).

Selon une variante particulièrement intéressante de l'invention, le groupement phényle des composés de formule (I) est substitué par un groupement R₁ tel que défini dans la formule (I) situé en position *para.*

Les groupements R₁ préférés selon l'invention sont les groupements choisis parmi trifluorométhoxy, 4-acétylphényle, 4-pyridyle, 3-pyridyle, N-pyrrolydinyle, 1-méthylpyrrol-3-yl, 3,6-dihydro-2H-pyridin-1-yl, cyclohexyle, 2-hydroxy-4-pyridyle, et 2-hydroxyphényle.

D'une façon très avantageuse R₁ représente un groupement choisi parmi trifluorométhoxy, 4-acétylphényle, cyclohexyle et 4-pyridyle.

D'une façon préférée q est un entier égal à zéro.

Selon une variante avantageuse de l'invention, m est un entier choisi parmi zéro et un.

De même d'une façon avantageuse pour les composés préférés de l'invention, p est un entier choisi parmi zéro et un.

Le groupement R₇ préféré selon l'invention est le groupement phényle.

Les groupements R₈ préférés selon l'invention sont les groupements choisis parmi carboxy et aminocarbonyle.

Les isomères, ainsi que les sels d'addition à un acide ou à une base pharmaceutiquement acceptable, des variantes et des composés préférés font partie intégrante de l'invention.

L'invention concerne aussi les sels pharmaceutiquement acceptables des composés de formule (I). Une revue des sels pharmaceutiquement acceptables est notamment décrite dans *J. Pharm. Sci.,* **1977,** 66, 1-19.

Les acides pharmaceutiquement acceptables signifient les acides non toxiques organiques ou minéraux. Parmi les acides pharmaceutiquement acceptables, on peut citer à titre non limitatif les acides chlorhydrique, bromhydrique, sulfurique, phosphonique, nitrique, acétique, trifluoroacétique, lactique, pyruvique, malonique, succinique, glutarique, fumarique, tartrique, maléïque, citrique, ascorbique, oxalique, méthane sulfonique, camphorique, benzoique, toluènesulfonique, etc...

Les bases pharmaceutiquement acceptables signifient les bases non toxiques organiques ou minérales.
Parmi les bases pharmaceutiquement acceptables, on peut citer à titre non limitatif l'hydroxyde de sodium, l'hydroxyde de potassium, l'hydroxyde de lithium, l'hydroxyde de calcium, la triéthylamine, la tertbutylamine, la 2-diéthylaminoéthanol, l'éthanolamine, l'éthylènediamine, la dibenzyléthylènediamine, la piperidine, la pyrrolidine, la morpholine, la piperazine, la benzylamine, l'arginine, la lysine, l'histidine, la glucamine, la glucosamine, les hydroxides d'ammonium quaternaire, etc...

D'une façon générale on comprend par isomères des composés de l'invention, les isomères optiques tels que les énantiomères et les diastéréoisomères. Plus particulièrement, les formes énantiomères pures des composés de l'invention peuvent être séparées à partir des mélanges d'énantiomères qui sont mis à réagir avec un agent libérable de dédoublement des racémiques, ledit agent existant quant à lui sous la forme d'un énantiomère pur, permettant d'obtenir les diastéréoisomères correspondants. Ces diastéréoisomères sont ensuite séparés selon les techniques de séparation bien connues de l'homme de l'art, telles que la cristallisation ou la chromatographie, puis l'agent de dédoublement est éliminé en utilisant les techniques classiques de la chimie organique, pour conduire à l'obtention d'un énantiomère pur. D'une autre façon les formes énantiomères pures des composés de l'invention peuvent être séparées par chromatographie sur colonne chirale.

Les composés de l'invention qui sont présents sous la forme d'un mélange de diastéréoisomères, sont isolés sous forme pure par l'utilisation des techniques classiques de séparation telles que les chromatographies.

Dans certains cas particuliers, le procédé de séparation des composés de l'invention peut conduire à la formation prédominante d'un énantiomère ou d'un diastéréoisomère par rapport à l'autre.

L'invention s'étend également au procédé de préparation des composés de formule (I). Plus particulièrement les composés de formule (I) peuvent être obtenus à partir des composés de formule (II) : 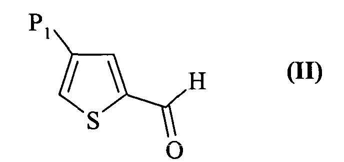 dans laquelle P₁ représente un atome d'halogène ou un groupement triflate,
composés de formule (II) qui sont soumis à des conditions d'oxydation en présence, par exemple, de nitrate d'argent en milieu basique et polaire, pour donner les composés de formule (III) : 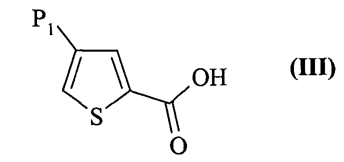 dans laquelle P₁ est tel que défini précédemment,
composés de formule (III) qui sont éventuellement transformés en chlorures d'acide correspondant (IV) par action du chlorure d'oxalyle par exemple, 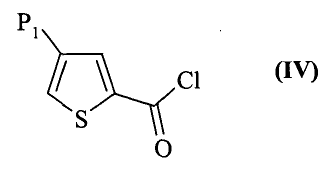 dans laquelle P₁ est tel que défini précédemment,
ou qui sont traités directement, dans des conditions de couplage peptidique en présence d'un agent de couplage et dans un milieu basique, avec un composé de formule (V) : 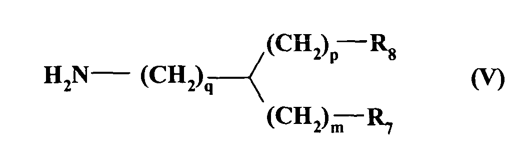 dans laquelle R₇, R₈, m, p et q ont les mêmes significations que dans les composés de formule (I),
pour conduire aux composés de (VI) : 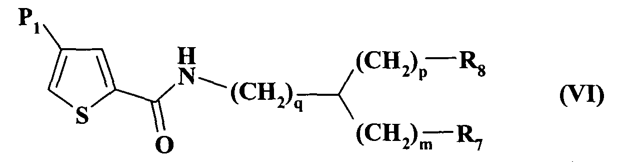 dans laquelle P₁, R₇, R₈, m, p et q sont tels que définis précédemment,
composés de formule (VI) qui sont :
- soit mis à réagir, dans des conditions basiques de couplage au palladium, avec un composé de formule (VII) : 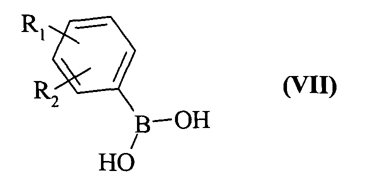 dans laquelle R₁ et R₂ ont les mêmes significations que dans les composés de formule (I), pour conduire aux composés de formule (I) ;
- soit traités avec de l'hexaméthyldiétain, en présence d'un catalyseur au palladium, pour conduire aux composés de formule (VIII) : 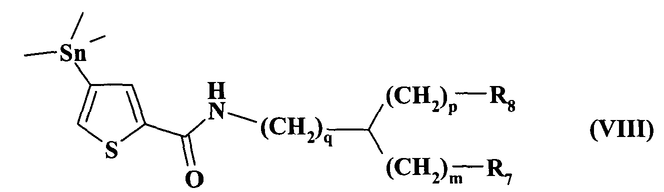 dans laquelle R₇, R₈, m, p et q sont tels que définis précédemment,
   composés de formule (VIII) qui sont mis à réagir :
   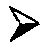 avec un composé de formule (IX) : 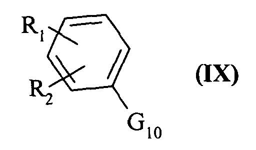 dans laquelle R₁ et R₂ ont les mêmes significations que dans la formule (I) et G₁₀ représentent un atome d'halogène choisi parmi chlore et brome ou un groupement triflate,
   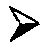 soit en présence de triphénylphosphine arsenic et d'un catalyseur au palladium, dans le cas où G₁₀ représente un groupement triflate,
   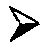 soit en présence d'un composé halogénocuivrique tel que CuBr₂ et d'un catalyseur au palladium, dans des conditions de solvant polaire, dans le cas où G₁₀ représente un atome d'halogène,
   pour conduire également aux composés de formule (I) ;
- soit traités par du bis(pinacolato)di-borane suivi par une réaction d'oxydation pour conduire aux composés de formule (VIa) : 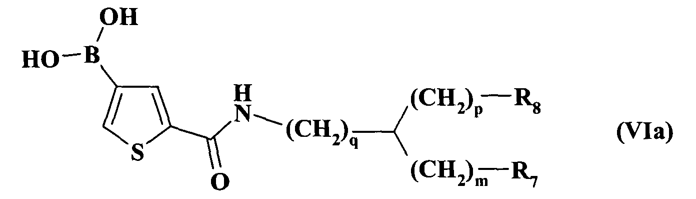 dans laquelle P₁, R₇, R₈, m, p et q sont tels que définis précédemment,
   composés de formule (VIa) qui sont mis à réagir dans des conditions basiques de couplage au palladium avec un composé de formule (IX) : 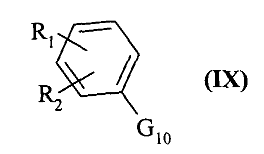 dans laquelle R₁, R₂ et G₁₀ sont tels que définis précédemment, pour conduire également aux composés de formule (I) ;
- soit mis à réagir avec un composé de formule (IXa): 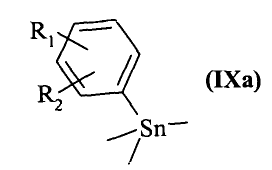 dans laquelle R₁, et R₂ sont tels que définis dans la formule (I),
   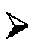 soit en présence de triphénylphosphine arsenic et d'un catalyseur au palladium, dans le cas où P₁ dans les composés de formule (VI) représente un groupement triflate,
   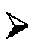 soit en présence d'un composé halogénocuivrique tel que CuBr₂ et d'un catalyseur au palladium, dans des conditions de solvant polaire, dans le cas où P₁ dans les composés de formule (VI) représente un atome d'halogène,
   pour conduire également aux composés de formule (I) ;
composés de formule (I) dans le cas particulier où ils représentent des composés de formule (I/a) dans lesquels R₂ représente un atome d'hydrogène, R₁ représente un groupement hydroxy ou un atome d'halogène (Hal) et R₇, R₈, m, p et q ont les mêmes significations que dans la formule (1) : 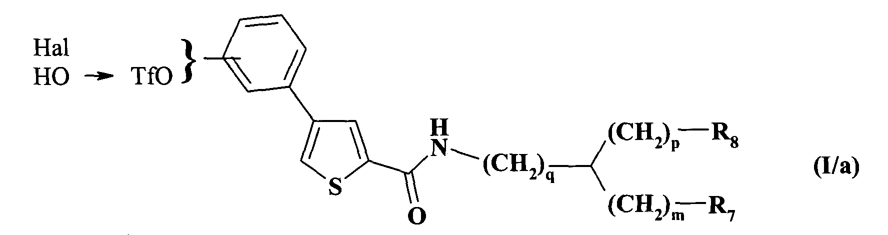 composés de formule (I/a) qui peuvent alors être traités préalablement par de l'anhydride trifluorométhanesulfonique en présence d'une base forte, dans le cas où R₁ représente un groupement hydroxy, pour obtenir le dérivé activé triflate,
lesdits composés portant un groupement R₁ prenant la définition halogène ou triflate pouvant alors
o soit être mis à réagir en condition basique et en présence d'un catalyseur au palladium avec un composé de formule (X) : 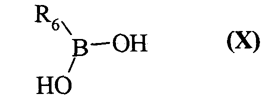 dans laquelle R₆ est tel que défini dans la formule (I), c'est à dire qu'il représente un groupement choisi parmi aryle, cycloalkyle, et un hétérocycle,
   pour conduire aux composés de formule (I/b), cas particulier des composés de formule (I) : 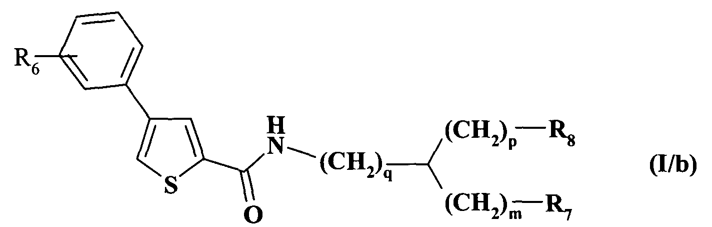 dans laquelle R₆, R₇, R₈, m, p et q sont tels que définis précédemment,
o soit être traités par du bis(pinacolato)di-borane suivi par d'une réaction d'oxydation, pour conduire aux composés de formule (XI) : 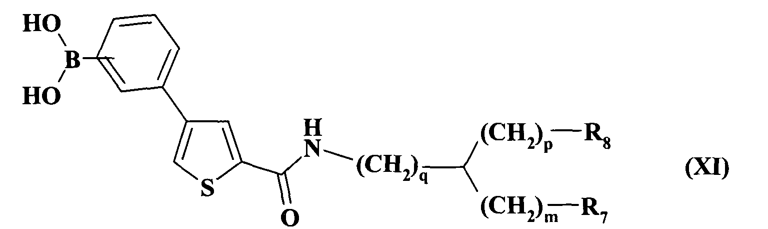 dans laquelle R₇, R₈, m, p et q sont tels que définis précédemment, composés de formule (XI) qui sont mis à réagir en condition basique et en présence d'un catalyseur au palladium avec un composé de formule (Xa) :

   R₆-P₂ **(Xa)**

   dans laquelle R₆ est tel que défini dans la formule (I), c'est à dire qu'il représente un groupement choisi parmi aryle, cycloalkyle, et un hétérocycle, et P₂ représente un atome d'halogène ou un groupement triflate,
   pour conduire également aux composés de formule (I/b) ;
o soit être traités avec de l'hexaméthyldiétain, en présence d'un catalyseur au palladium, pour conduire aux composés de formule (XIa) : 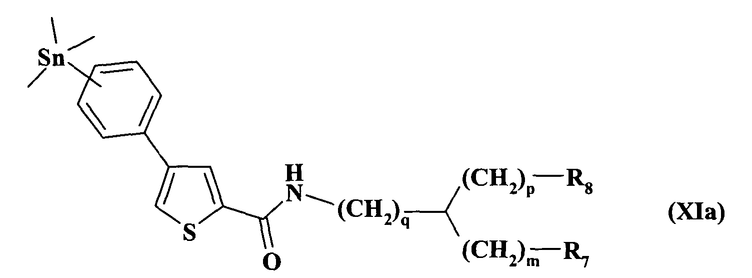 dans laquelle R₇, R₈, m, p et q sont tels que définis précédemment,
   composés de formule (XIa) qui sont mis à réagir avec un composé de formule (Xa) tel que défini précédemment :

   R₆-P₂ **(Xa)**

   dans laquelle R₆ est tel que défini dans la formule (I), P₂ représente un atome d'halogène ou un groupement triflate,
   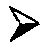 soit en présence de triphénylphosphine arsenic et d'un catalyseur au palladium, dans le cas où P₂ représente un groupement triflate,
   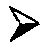 soit en présence d'un composé halogénocuivrique tel que CuBr₂ et d'un catalyseur au palladium, dans des conditions de solvant polaire, dans le cas où P₂ représente un atome d'halogène,
   pour conduire également aux composés de formule (I/b) ;
o soit être mis à réagir avec un composé de formule (Xb):

   R₆-SnMe₃ **(Xb)**

   dans laquelle R₆ est tel que défini précédemment,
   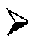 soit en présence de triphénylphosphine arsenic et d'un catalyseur au palladium, dans le cas où les composés de formule (I/a) comportent un groupement triflate,
   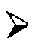 soit en présence d'un composé halogénocuivrique tel que CuBr₂ et d'un catalyseur au palladium, dans des conditions de solvant polaire, dans le cas où les composés de formule (I/a) comportent un atome d'halogène,
   pour conduire également aux composés de formule (I/b) ;
o soit être mis à réagir dans des conditions de couplage au palladium, en milieu basique, avec un composé de formule (XII) :

   R_{6'}-H **(XII)**

   dans laquelle R₆, représente un hétérocycle azoté éventuellement substitué par un ou plusieurs groupements tels qu'ils sont définis pour les substituants du groupement R₆ au sein des composés de formule (I),
pour conduire aux composés de formule (I/c), cas particulier des composés de formule (I) : 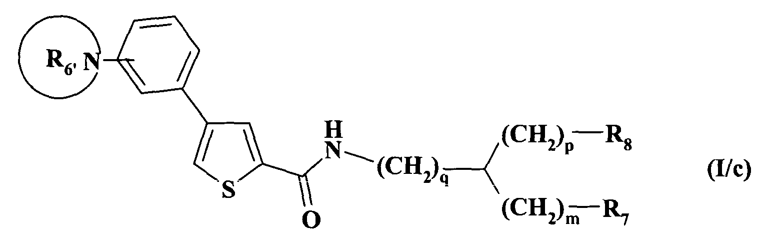 dans laquelle R₇, R₈, m, p et q sont tels que définis précédemment, et R₆, représente un hétérocycle azoté éventuellement substitué tel que défini dans la formule (I),
les composés (I/a) à (I/c) formant ensemble des composés de l'invention, qui sont purifiés, le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

Les composés de formule (II), (V), (VII), (IX), (X) et (Xa) sont soit des composés commerciaux, soit obtenus selon des méthodes connues de la synthèse organique aisément accessibles et compréhensibles à l'homme du métier.

Selon une variante de l'invention les composés de formule (I) peuvent également être obtenus par un second procédé de préparation caractérisé en ce qu'on utilise comme produit de départ un composé de formule (II) : 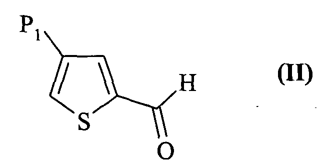 dans laquelle P₁ représente un atome d'halogène ou un groupement triflate, composés de formule (II) qui sont soumis à des conditions d'oxydation en présence, par exemple, de nitrate d'argent en milieu basique et polaire, pour donner les composés de formule (III) : 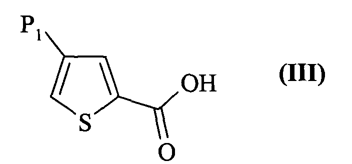 dans laquelle P₁ est tel que défini précédemment,
composés de formule (III) dont la fonction acide est estérifiée par action d'un alcool en présence d'un acide fort, pour conduire aux composés de formule (XX) : 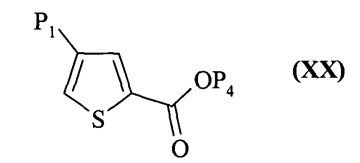 dans laquelle P₁ est tel que défini précédemment, et P₄ représente un groupement (C₁-C₄)alkyle linéaire ou ramifié,
composés de formule (XX) qui sont :
- soit mis à réagir, dans des conditions basiques de couplage au palladium, avec un composé de formule (VII) : 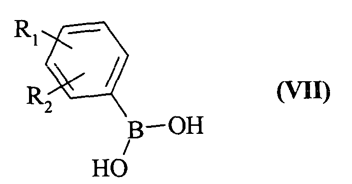 dans laquelle R₁ et R₂ ont les mêmes significations que dans les composés de formule (I), pour conduire aux composés de formule (XXa) : 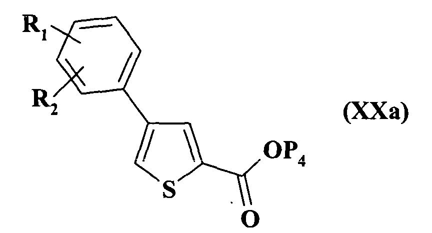 dans laquelle R₁, R₂ et P₄ sont tels que définis précédemment,
- soit traités avec de l'hexaméthyldiétain, en présence d'un catalyseur au palladium, pour conduire aux composés de formule (XXI) : 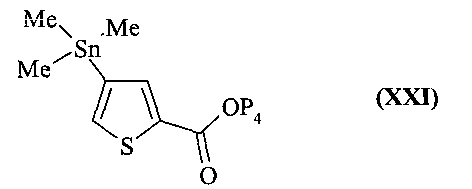 dans laquelle P₄ est tel que défini précédemment,
   composés de formule (XXI) qui sont mis à réagir :
   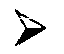 avec un composé de formule (IX) : 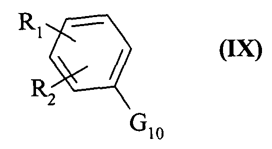 dans laquelle R₁ et R₂ ont les mêmes significations que dans la formule (I) et G₁₀ représentent un atome d'halogène choisi parmi chlore et brome ou un groupement triflate,
   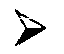 soit en présence de triphénylphosphine arsenic et d'un catalyseur au palladium, dans le cas où G₁₀ représente un groupement triflate,
   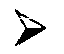 soit en présence d'un composé halogénocuivrique tel que CuBr₂ et d'un catalyseur au palladium, dans des conditions de solvant polaire, dans le cas où G₁₀ représente un atome d'halogène,
   pour conduire également aux composés de formule (XXa) telle que décrite précédemment ;
- soit traités par du bis(pinacolato)di-borane, suivi d'une réaction d'oxydation, pour conduire aux composés de formule (XXII) : 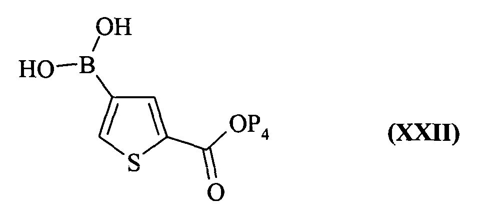 dans laquelle P₄ est tel que défini précédemment,
   composés de formule (XXII) qui sont mis à réagir dans des conditions basiques de couplage au palladium avec un composé de formule (IX) tel que décrit précédemment pour conduire également aux composés de formule (XXa), tels que définis précédemment ;
- soit mis à réagir avec un composé de formule (IXa), (lesdits composés de formule (IXa) étant obtenus par traitement des composés de formule (IX) tels que définis précédemment, avec de l'hexaméthyldiétain en présence d'un catalyseur au palladium) : 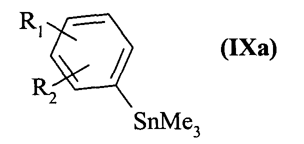 dans laquelle R₁, et R₂ sont tels que définis dans la formule (I),
   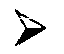 soit en présence de triphénylphosphine arsenic et d'un catalyseur au palladium, dans le cas où P₁ dans les composés de formule (XX) représente un groupement triflate,
   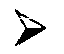 soit en présence d'un composé halogénocuivrique tel que CuBr₂ et d'un catalyseur au palladium, dans des conditions de solvant polaire, dans le cas où P₁ dans les composés de formule (XX) représente un atome d'halogène,
   pour conduire également aux composés de formule (XXa) tels que définis précédemment ;
composés de formule (XXa) qui sont saponifiés dans des conditions d'hydrolyse basique, pour conduire aux composés de formule (XXb) : 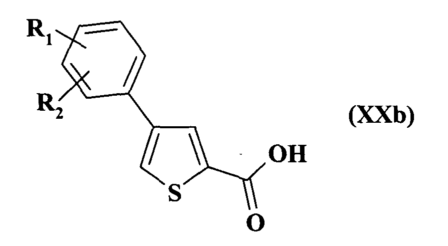 dans laquelle R₁ et R₂ sont tels que définis dans la formule (I),
composés de formule (XXb) qui sont soit transformés préalablement en chlorure d'acide correspondant par action du chlorure d'oxalyle, soit traités directement, dans des conditions de couplage peptidique en présence par exemple d'en agent de couplage et dans un milieu basique, avec un composé de formule (V) : 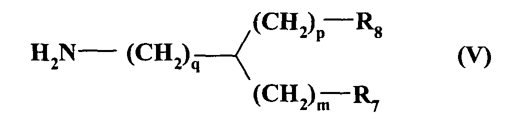 dans laquelle R₇, R₈, m, p et q ont les mêmes significations que dans les composés de formule (I),
pour conduire aux composés de formule (I): 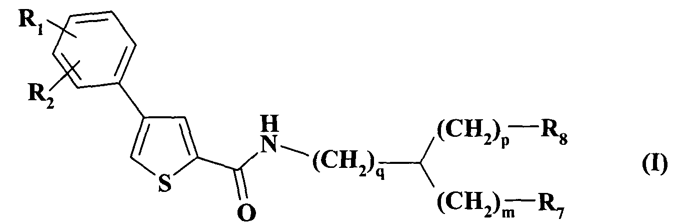 composés de formule (I), qui sont purifiés, le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

Les composés de la présente invention, de par leurs propriétés pharmacologiques d'inhibiteurs de la MMP-12, sont utiles dans la prévention et le traitement des pathologies respiratoires telles que les broncho-pneumopathies chroniques obstructives (BPCO), l'emphysème, les bronchites chroniques, les inflammations pulmonaires chroniques, l'asthme, la mucoviscidose, la détresse respiratoire aiguë (ARDS), les allergies respiratoires dont la rhinite allergique, ainsi que les maladies liées à la production de TNFα incluant les maladies pulmonaires fibrotiques sévères, la sarcoïdose pulmonaire, et la silicose. Les composés de la présente invention montrent aussi, à un niveau moindre, une activité inhibitrice de la métalloprotéinase-13 (MMP-13) les rendant potentiellement utiles pour le traitement des pathologies impliquant cette enzyme, telles que le cancer, l'ostéoporose, l'ostéoarthrite, l'arthrite, l'arthrite rhumatoïde, l'athéorosclérose, la sclérose en plaques, l'insuffisance cardiaque.

D'une façon avantageuse les composés de la présente invention sont utiles pour la prévention et le traitement des broncho-pneumopathies chroniques obstructives, de l'emphysème et des bronchites chroniques.

Plus particulièrement les composés de la présente invention sont utiles pour le traitement de l'emphysème lié au tabagisme.

Selon une variante de l'invention, les composés de formule (I) sont utiles pour la prévention et le traitement de l'asthme.

La présente invention a également pour objet les compositions pharmaceutiques renfermant comme principe actif au moins un composé de formule (I), un de ses isomères ou un de ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per- ou trans-cutanée, intravaginale, rectale, nasale, perlinguale ou respiratoire.

Les compositions pharmaceutiques selon l'invention pour les injections parentérales comprennent notamment les solutions stériles aqueuses et non aqueuses, les dispersions, les suspensions ou émulsions ainsi que les poudres stériles pour la reconstitution des solutions ou dispersions injectables.

Les compositions pharmaceutiques selon l'invention, pour les administrations orales solides, comprennent notamment les comprimés simples ou dragéifiés, les comprimés sublinguaux, les sachets, les gélules, les granules, et pour les administrations liquides orales, nasales ou buccales, comprennent notamment les émulsions, les solutions, les suspensions, les gouttes, les sirops et les aérosols.

Les compositions pharmaceutiques selon l'invention, pour les administrations par voie respiratoire, comprennent notamment des compositions sous forme de solutions pour aérosols ou de poudres pour inhalateurs. Lorsque les compositions sont des aérosols, pour l'usage d'aérosols liquides, les compositions peuvent être des solutions stériles stables ou des compositions solides dissoutes au moment de l'emploi dans de l'eau stérile apyrogène, dans du sérum physiologique ou tout autre véhicule pharmaceutiquement acceptable. Pour l'usage sous forme d'aérosols secs destinés à être directement inhalés, le principe actif est éventuellement finement divisé ou micronisé, et associé à un diluant ou véhicule inerte solide hydrosoluble.

Les compositions pharmaceutiques pour l'administration rectale sont préférentiellement des suppositoires, et celles pour l'administration per- ou trans-cutanée comprennent notamment les poudres, les aérosols, les crèmes, les pommades, les gels, et les patchs.

Les compositions pharmaceutiques citées précédemment illustrent l'invention mais ne la limitent en aucune façon.

Parmi les excipients ou véhicules inertes, non toxiques, pharmaceutiquement acceptables, on peut citer à titre indicatif et non limitatif les diluants, les solvants, les conservateurs, les agents mouillants, les émulsifiants, les agents dispersants, les liants, les agents gonflants, les agents désintégrants, les retardants, les lubrifiants, les absorbants, les agents de suspension, les colorants, les aromatisants, etc...

La posologie utile varie selon l'âge et le poids du patient, la voie d'administration, la composition pharmaceutique utilisée, la nature et la sévérité de l'affection, et la prise de traitements associés éventuels. La posologie s'échelonne de 1 mg à 1000 mg en une ou plusieurs prises par jour.

Les exemples suivants illustrent l'invention mais ne la limitent en aucune façon.

Les produits de départ utilisés sont des produits commerciaux ou des produits préparés selon des modes opératoires connus à partir de composés commerciaux ou connus par l'homme du métier. Les différentes préparations conduisent à des intermédiaires de synthèse utiles pour la préparation des composés de l'invention.

Les structures des composés décrits dans les exemples et dans les préparations ont été déterminées selon les techniques spectrophotométriques usuelles (infrarouge (IR), résonance magnétique nucléaire (RMN), spectrométrie de masse (SM) dont électrospray (ES), ...) et la pureté a été déterminée par chromatographie liquide à haute performance (HPLC).

Abréviations utilisées dans les modes opératoires :
- TOTU : O-[(éthoxycarbonyl)cyanométhylamino]-*N*-*N-N*'-*N*'-tetraméthyl uronium fluoroborate ;
- DME : 1,2-diméthoxyéthane (ou éthylène glycol diméthyl éther)
- TFA : acide trifluoroacétique
- HATU :O-(7-azabenzotriazol-1-yl)-*N*,*N*,*N*',*N*'-tétraméthyluronium hexafluorophosphate
- tBME : tert-butyl méthyl éther

### Préparation 1 : Acide 4-[4-(trifluorométhoxy)phényl]thiophèn-2-carboxylique

### Stade 1 : 4-[4-(Trifluorométhoxy)phényl]thiophèn-2-carbaldéhyde

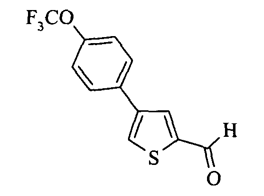

A une solution de 12,3 g de 4-bromothiophène-2-carbaldéhyde et 20,0 g d'acide [4-(trifluorométhoxy)phényl]-boronique (1,2 équivalents) dans 70 ml de DME dégazé sont additionnés 84,9 ml (2,1 équivalents) d'une solution 2,0 M de phosphate de potassium et 2,8 g (0,03 équivalent) de tétrakis(triphénylphosphine)palladium(0). Le milieu réactionnel est agité 3 heures à 80°C puis concentré sous pression réduite. Le résidu obtenu est repris par de l'acétate d'éthyle. La solution est alors filtrée sur célite, lavée à l'eau, séchée sur sulfate de sodium, filtrée puis concentrée sous pression réduite. Une chromatographie sur gel de silice du résidu (cyclohexane/acétate d'éthyle : 9/1) permet d'isoler 15,05 g du produit attendu.
Rendement : 68 %
RMN ¹H (CDCl₃) δ (ppm) : 10,0 (s, 1H), 8,0 (s, 1H), 7,80 (s, 1H), 7,55 (m,2H), 7,25 (m, 2H)

### Stade 2 : Acide 4-[4-(trifluorométhoxy)phényl]thiophèn-2-carboxylique

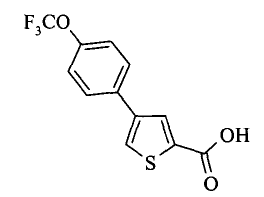

A une solution de 15,05 g du composé obtenu au stade 1 dans 200 ml d'éthanol sont additionnés 37,6 g (4 équivalents) de nitrate d'argent et 44,2 ml (8 équivalents) d'une solution aqueuse 1,0 M de soude. Le milieu réactionnel est agité 2 heures à 40°C, puis filtré sur célite et concentré sous pression réduite. La phase aqueuse est lavée par une solution aqueuse 1,0 M d'acide chlorhydrique, extraite à l'acétate d'éthyle, séchée sur sulfate de sodium, filtrée et concentrée sous pression réduite, permettant d'obtenir 15,514 g d'une poudre beige correspondant au produit attendu.
Rendement : 97,4 %
SM : MH- 287

### Préparation 2 : (2S)-2-Amino-N cyclohexyl-2-phénylacétamide

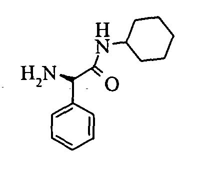

### Stade 1 : (2S)-tert-Butoxy-2-(cyclohexylamino)-2-oxo-1-phényléthylcarbamate

A une solution de 300 mg d'acide (*2S*)-[(tert-butoxycarbonyl)amino](phényl)acétique dans 5 ml de diméthylformamide anhydre sont ajoutés 0,164 ml de cyclohexylamine (1,2 équivalents), 0,548g de O-[(éthoxycarbonyl)cyanométhyleneamino]-*N*,*N*,*N*',*N*'-tétraméthyluronium (TOTU) et 830 µl de *N*-éthyl-*N*,*N*-diisopropylamine. Le milieu réactionnel est agité pendant 3 heures à température ambiante puis concentré sous pression réduite. Le résidu obtenu est solubilisé dans l'acétate d'éthyle (30 ml), lavé à l'eau (30 ml), séché sur sulfate de sodium, filtré et concentré sous pression réduite. Une chromatographie sur gel de silice (70/30 : cyclohexane/acetate d'éthyle) du résidu permet d'isoler 258 mg du produit attendu.
Rendement : 65 %
SM : MH⁺ 333

### Stade 2 : (2S)-2 Amino-N cyclohexyl-2 phénylacétamide

A une solution de 258 mg du produit obtenu au stade 1 dans 4 ml de dichlorométhane anhydre sont ajoutés 0,297 ml d'acide trifluoroacétique à 0°C . Le milieu réactionnel est agité pendant 17 heures à température ambiante, lavé à l'eau (30 ml) puis avec une solution saturée d'hydrogénocarbonate de sodium (30 ml), séché sur sulfate de sodium, filtré et concentré sous pression réduite. Une recristallisation du résidu dans l'éther diisopropylique permet d'isoler 219 mg du produit attendu.
Rendement : 99 %
RMN ¹H (CDCl₃) δ (ppm) : 7,30 (m, 5H), 6,90 (m, 1H), 4,50 (m, 1H), 4,10 (m, 2H), 3,80 (m, 1H), 2,50 (m, 1H), 1,60 (m, 12H)
SM : MH⁺ 233
HPLC : 97,1 %

### Préparation 3 : (3S)-3-Amino-4-phényl-butanoate d'éthyle

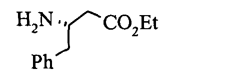

A une solution de 0,5 g d'acide (*3S*)-3-[(*tert*-butoxycarbonyl)amino]-4-phénylbutanoique dans 10 ml d'éthanol est additionné 1,0 ml d'acide sulfurique concentré (10 vol). Le milieu réactionnel est agité 17 heures à reflux. La solution est alors concentrée sous pression réduite et basifiée avec une solution d'hydrogénocarbonate de sodium saturée jusqu'à pH 8. La solution obtenue est extraite avec de l'acétate d'éthyle et la phase organique est séchée sur sulfate de sodium puis filtrée pour obtenir 0,286 g du produit attendu après évaporation sous pression réduite.
Rendement : 77 %
RMN ¹H (DMSO) δ (ppm) : 7,30 (m, 2H), 7,20 (m, 3H), 4,0 (q, 2H), 3,20 (m, 1H), 2,70 (m, 2H), 2,30 (m, 2H), 1,50 (s, 2H), 1,10 (t, 3H)
SM: MH⁺ 208
HPLC : 100 %

### Préparation 4 : (4R)-4-Amino-5-phényl-pentanoate de méthyle

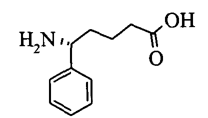

### Stade 1 : (3R)-3-[(tert-Butoxycarbonyl)amino]-3 phényl-propanoate de méthyle

A une solution de 0,31 g d'acide (3R)-3-[(tert-butoxycarbonyl)amino]-3-phénylpropanoique, préparé selon la méthode décrite dans *Tetrahedron,* **1997**, 53, 12867-12874, dans un mélange de 6 ml d'éther éthylique et 6 ml de méthanol sont additionnés 1,9 ml de triméthylsilyldiazométhane en solution 2,0 M dans le cyclohexane. Le milieu réactionnel est agité 1 heure à température ambiante puis hydrolysé avec 100 µl d'acide acétique et concentré sous pression réduite. Le produit brut est trituré dans un mélange cyclohexane/acétate d'éthyle(1/2) et filtré pour conduire à 0,386 g du produit désiré.
Rendement : 77 %
RMN ¹H (DMSO) δ (ppm) : 7,40 (m, 1H), 7,30 (m, 5H), 4,40 (m, 1H), 3,60 (s, 3H), 2,30 (m, 2H), 1,80 (m, 2H), 1,40 (s, 9H)
SM : MH⁺ 294
HPLC : 100 %

### Stade 2 : (4R)-4 Amino-5 phényl-pentanoate de méthyle

A une solution de 0,1 g du produit obtenu au stade 1 dans 2,0 ml de méthanol sont additionnés à 0°C 1,62 ml (10 équivalents) d'une solution d'acide chlorhydrique 2,1 M dans le méthanol. Le milieu réactionnel est agité 1 heure à température ambiante et concentré sous pression réduite pour conduire à 73 mg du produit désiré.
Rendement : 93 %
RMN ¹H (DMSO) δ (ppm) : 8,90 (m, 3H), 7,40 (m, 5H), 4,40 (m, 1H), 3,60 (s, 3H), 2,30 (m, 2H), 2,0 (m, 2H)
SM : MH⁺ 194

### Préparation 5 : Acide (4-cyclohexyl)phényl-boronique

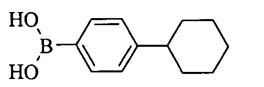

### Stade 1 : 4-[4-(Pinacolboro)phényl]cyclohexyle

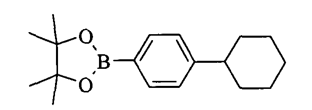

A une solution de 0,3 g de bromophénylcyclohexyle dans 5 ml de 1,4-dioxane dégazé sont ajoutés sous azote 0,382 g de bis(pinacolate)di-bore (1,2 équivalents), 27,5 mg de dichloro[[1,1']-bis(diphénylphosphino)ferrocène]palladium (II) (0,03 équivalents), 41,7 mg de [1,1']-bis(diphénylphosphino)ferrocène (0,06 équivalents) et 0,369 g d'acétate de potassium (3 équivalents). Le milieu réactionnel est agité pendant 17 heures à 80°C, dilué avec de l'acétate d'éthyle (100 ml), lavé à l'eau (3x60 ml), séché sur sulfate de sodium et concentré sous pression réduite. Une chromatographie sur gel de silice (heptane/acétate d'éthyle : 95/5) du résidu permet d'obtenir 0,2 g du produit désiré.
Rendement : 56 %
RMN ¹H (DMSO) δ (ppm) : 7,60 (d, 2H), 7,20 (d, 2H), 1,70 (m, 5H), 1,40 (m, 4H), 1,30 (m, 12H)

### Stade 2 : Acide (4-cyclohexyl)phényl-boronique

A une solution de 0,103 g du composé obtenu au stade 1 précédent dans 3 ml d'acétone sont ajoutés 3 ml d'eau et 0,23 g de périodate de sodium (3,0 équivalents). Le milieu réactionnel est agité pendant 17 heures à 60°C puis concentré sous pression réduite. Le résidu obtenu est solubilisé avec de l'acétate d'éthyle (20 ml), lavé avec une solution d'acide chlorhydrique 1,0 M puis avec de l'eau (3x10 ml), séché sur sulfate de sodium, filtré puis concentré sous pression réduite. Une chromatographie sur gel de silice (dichlorométhane/méthanol : 90/10) du résidu permet d'obtenir 64 mg du produit désiré.
Rendement : 87 %
SM: MH⁻(+HCO₂H) 249
HPLC: 100 %

### Préparation 6 : 4-(4-Bromophényl)thiophène-2-carboxylate de méthyle

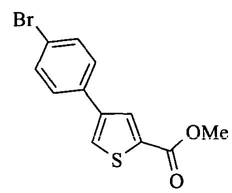

### Stade 1 : 4-(4-Nitrophényl)thiophène-2-carboxylate de méthyle

Le produit (1,94 g) est obtenu selon le procédé du stade 1 de la préparation 1 en utilisant comme substrats le 4-bromothiophène-2-carboxylate de méthyle et l'acide (4-nitrophényl)boronique.
Rendement : 78 %
RMN ¹H (CDCl₃) δ (ppm) : 3,92 (s, 3H), 7,75 (d, 2H), 7,82 (s, 1H), 8,12 (s, 1H), 8,30 (d, 2H)

### Stade 2 : 4-(4-Aminophényl)thiophène-2-carboxylate de méthyle

Une solution de 1,94 g du composé obtenu au stade 1 précédent dans 20 ml de méthanol contenant 194 mg de palladium sur charbon à 10% est agitée dans un autoclave pendant 6 heures à 50°C sous 10 bars d'hydrogène. Le milieu réactionnel est ensuite filtré sur célite et concentré sous pression réduite permettant d'obtenir 1,51 g du produit désiré.
Rendement : 88 %
RMN ¹H (DMSO) δ (ppm) : 3,82 (s, 3H), 5,22 (s, 2H), 6,60 (d, 2H), 7,42 (d, 2H), 7,90 (s, 1H), 8,05 (s, 1H)
SM : MH⁺ 234

### Stade 3 : 4-(4-Bromophényl)thiophène-2-carboxylate de méthyle

A une solution de 103 mg de produit obtenu lors du stade 2 précédent dans 1,5 ml d'eau est ajouté 0,6 ml d'acide bromhydrique concentré. Le milieu réactionnel est refroidi à 0°C, puis une solution de 35,5 mg de nitrite de sodium (1,1 équivalents) dans 0,5 ml d'eau est ajoutée goutte à goutte. Après 1 heure d'agitation à 0°C, une solution de 68 mg de bromure de cuivre dans 0,5 ml d'acide bromhydrique concentré est ajoutée goutte à goutte. Le milieu réactionnel est de nouveau agité pendant 1 heure à 0°C puis dilué avec de l'acétate d'éthyle (30 ml), lavé à l'eau (3x15 ml), lavé avec une solution saturée d'hydrogénocarbonate de sodium (15 ml) puis de nouveau à l'eau (15 ml). La phase organique est séchée sur sulfate de sodium, filtrée puis concentrée sous pression réduite. Une chromatographie sur gel de silice du résidu (cyclohexane/acétate d'éthyle : 95/5) permet d'isoler 52 mg du produit désiré.
Rendement : 40 %
RMN ¹H (CDCl₃) δ (ppm) : 3,92 (s, 3H), 7,45 (d, 2H), 7,55 (d, 2H), 7,65 (s, 1H), 8,05 (s, 1H)
HPLC : 91,4 %

### Exemple 1 : (2S)-{[4-(4-Trifluorométhoxyphényl)thièn-2-yl]carboxamido} (phényl)acétate d'éthyle

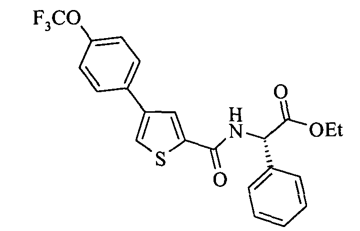

A une solution de 300 mg du composé obtenu dans la préparation 1 dans 6 ml de diméthylformamide anhydre sont ajoutés 246 mg de chlorhydrate de *(2S)*-amino(phényl)acétate d'éthyle (1,1 équivalents), 395 mg de O-(7-azabenzotriazol-1-yl)-*N*,*N*,*N'*,*N'*-tétraméthyluronium hexafluorophosphate (HATU) et 362 µl de *N*-éthyl-*N*,*N*-diisopropylamine. Le milieu réactionnel est agité pendant 17 heures à température ambiante puis hydrolysé. Le précipité formé est filtré, lavé à l'eau et enfin séché pendant une nuit pour conduire à 639 mg de produit attendu.
Rendement : 100 %
RMN ¹H (DMSO) δ (ppm) : 9,20 (d, 1H), 8,5 (s, 1H), 8,20 (s, 1H), 7,85 (d, 2H), 7,45 (m, 7H), 5,6 (d, 1H), 4,15 (m, 2H), 1,15 (t, 3H).
HPLC : 98,50 %

### Exemple 2 : Acide (2S)-({[4-(4-trifluorométhoxyphényl)thièn-2-yl)carboxamido} (phényl) acétique

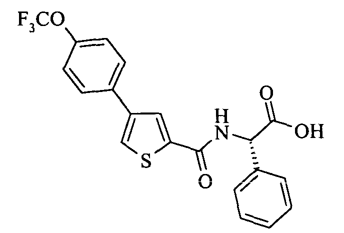

A une solution de 639 mg du composé obtenu dans l'exemple 1 dans 22 ml d'un mélange éthanol/eau (1/1) sont ajoutés 170 mg d'hydroxyde de lithium (5 équivalents) et 200 µl de diméthylformamide. Le milieu réactionnel est agité pendant 1 nuit à température ambiante puis concentré sous pression réduite. Le solide obtenu est repris dans l'eau et acidifié avec une solution d'acide chlorhydrique 1,0 M jusqu'à pH 1. Le précipité formé est alors filtré, lavé à l'eau puis séché pendant une nuit pour conduire à 377 mg.
Rendement : 64 %
RMN ¹H (DMSO) δ (ppm) : 13,0 (bs, 1H), 8,94 (s, 1H), 8,49 (s, 1H), 8,17 (s, 1H), 7,84 (d, 2H), 7,43 (m, 7H), 5,44 (s, 1H)
SM : MH⁺ 422
HPLC : 98,4 %

### Exemple 3 : (2R)-{[4-(4-Trifluorométhoxyphényl)thièn-2-yl]carboxamido} (phényl)acétate d'éthyle

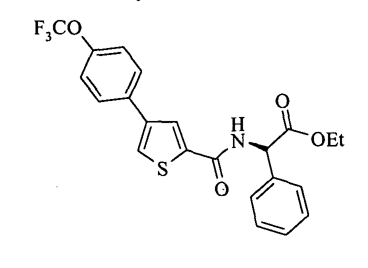

Le produit (200 mg) est obtenu selon le procédé de l'exemple 1 en utilisant comme substrat le chlorhydrate de (*2R*)-amino(phényl)acétate d'éthyle.
Rendement : 44 %
SM : MH⁺ 436
HPLC : 98,96 %

### Exemple 4 : Acide (2R)-{[4-(4-trifluorométhoxyphényl)thièn-2-yl]carboxamido} (phényl) acétique

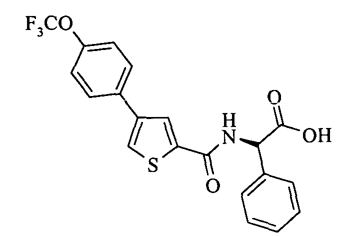

Le produit (121 mg) est obtenu selon le procédé de l'exemple 2 en utilisant comme substrat le composé obtenu dans l'exemple 3.
Rendement : 65 %
RMN ¹H (DMSO) δ (ppm) : 13,01 (bs, 1H), 9,075 (d, 1H), 8,54 (s, 1H), 8,19 (s, 1H), 7,83 (d, 2H), 7,42 (m, 7H), 5,57 (d, 1H)
SM : MH⁺ 422
HPLC : 99,0 %

### Exemple 5 : 3-{[4-(4-Trifluorométhoxyphényl)thièn-2-yl]carboxamido)-3-phényl-propanoate d'éthyle

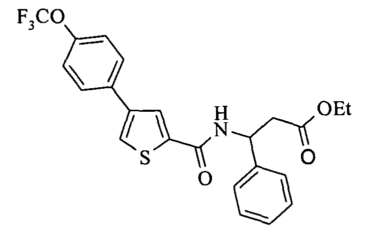

Le produit (283 mg) est obtenu selon le procédé de l'exemple 1 en utilisant comme substrat le chlorhydrate d'amino(phényl)propanoate d'éthyle.
Rendement : 59 %
SM : MH⁺ 464
HPLC : 96.69 %

### Exemple 6 : Acide {[4-(4-Trifluorométhoxyphényl)thièn-2-yl]carboxamido}(phényl) propanoique

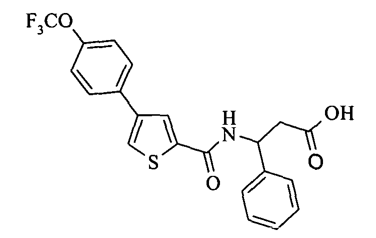

Le produit (198 mg) est obtenu selon le procédé de l'exemple 2 en utilisant comme substrat le composé obtenu dans l'exemple 5.
Rendement : 75 %
RMN ¹H (DMSO) δ (ppm) : 9,03 (d, 1H), 8,29 (s, 1H), 8,15 (s, 1H), 7,82 (d, 2H), 7,46 (d, 2H), 7,41 (d, 2H), 7,34 (t, 2H), 7,25 (t, 1H), 5,40 (q, 1H), 2,83 (m, 2H)
SM : MH⁺ 436
HPLC : 99,3 %

### Exemples 7 et 8 : Acide (2S)-{[4-(4-trifluorométhoxyphényl)thièn-2-yl] carboxamido}(phényl) propanoique et Acide (2R) {[4-(4-Trifluorométhoxyphényl)thièn-2-yl]carboxamido} (phényl) propanoique

Les produits (60,9 mg et 50,2 mg) sont obtenus à partir du mélange racémique de l'exemple 6 après une séparation chirale sur HPLC préparative selon les conditions suivantes :
Colonne : Chiralpack® AD-H 20 x 250 mm, 5 µm, température : ambiante, détection UV : 214 nm, débit : 20 mL/min,
Phase mobile : T = 0, B = 70 % d'hexane, A = 30 % d'isopropanol contenant 0.2 % diéthylamine (v/v) ; T = 30min, B = 30 %, A =70 % (v/v).

### Exemple 7 : Acide (2S)-{[4-(4-Trifluorométhoxyphényl]thièn-2 yl]carboxamido}(phényl) propanoique

Rendement : 31 %
RMN ¹H (DMSO) δ (ppm) : 9,16 (bs, 1H), 8,29 (s; 1H), 8,14 (s, 1H), 7,82 (d, 2H), 7,45 (d, 2H), 7,40 (d, 2H), 7,33 (t, 2H), 7,24 (t, 1H), 5,38 (q, 1H), 2,79 (m, 2H)
SM : MH⁺ 436
HPLC : 99,21 %

### Exemple 8 : Acide (2R)-{[4-(4-Trifluorométhoxyphényl)thièn-2 yl]carboxamido}(phényl) propanoique

Rendement : 26 %
RMN ¹H (DMSO) δ (ppm) : 12,51 (bs, 1H), 9,11 ( m, 1H), 8,29(s, 1H), 8,14 (s, 1H), 7,82 (d, 2H), 7,46 (d, 2H),7,41 ( d, 2H), 7,33 (t, 2H), 7,24 (t, 1H)
SM : MH⁺ 436
HPLC : 99,70 %

### Exemple 9: N-(3-amino-3-oxo-1-phénylpropyl)-4-(4-trifluorométhoxy phényl)thiophène-2-carboxamide

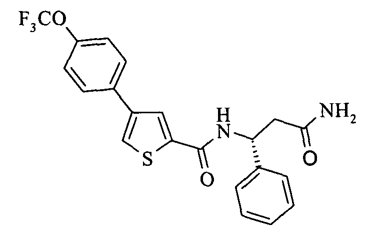

A une solution de 335 mg du composé obtenu dans l'exemple 8 dans 5 ml de tétrahydrofurane anhydre est additionné à 0 °C un large excès d'une solution aqueuse d'ammoniaque à 28%. Le bain glacé est retiré et l'ensemble est agité pendant une nuit à température ambiante. Le brut réactionnel est concentré sous pression réduite, hydrolysé et extrait au dichlorométhane. Les phases organiques sont réunies, séchées sur sulfate de sodium et concentrées. Une chromatographie sur gel de silice (70/30 : cyclohexane/acétated'éthyle) du résidu permet d'obtenir 33 mg du produit désiré.
Rendement : 15 %
RMN ¹H (DMSO) δ (ppm) : 9,11 (d, 1H), 8,29 (s, 1H), 8,14 (s, 1H), 7,82 (d, 2H), 7,46 (m, 2H), 7,41 (m, 5H), 7,20 (m, 1H), 6,80 (m, 1H), 5,40 (m,1H), 2,70 (m, 2H)
SM : MH⁻ 433
HPLC : 100 %

### Exemple 10 : 4-(4-Trifluorométhoxyphényl)-N-[(2S)-2-(cyclohexylamino)-2-phényléthanoyl]thiophène-2-carboxamide

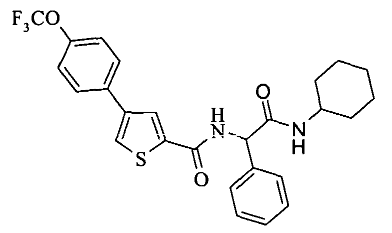

Le produit (265 mg) est obtenu selon le procédé du stade 1 de la préparation 2 en utilisant comme substrats les composés obtenus dans les préparations 1 et 2.
Rendement : 56 %
RMN ¹H (DMSO) δ (ppm) : 9,11 (m, 1H), 8,70 (s, 1H), 8,30 (m, 1H), 8,20 (s, 1H) 7,82 (d, 2H), 7,46 (m, 5H), 7,30 (m, 2H), 5,70 (d, 2H), 3,40 (m, 1H), 1,80 (m, 1H), 1,70 (m, 3H), 1,5 (m, 1H), 1,20 (m, 5H)
SM : MH⁺ 503
HPLC : 100 %

### Exemple 11 : (3S)-3-{[4-(4-Trifluorométhoxyphényl)thièn-2-yl]carboxamido}-4-phénylbutanoate d'éthyle

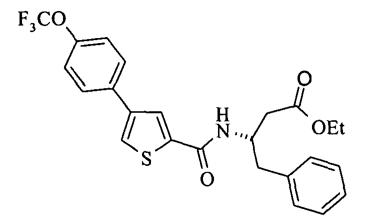

Le produit (191 mg) est obtenu selon le procédé de l'exemple 1 en utilisant comme substrat le produit de la préparation 3.
Rendement : 29 %
RMN ¹H (DMSO) δ (ppm) : 8,70 (m, 1H), 8,20 (s, 1H), 8,10 (s, 1H), 7,82 (d, 2H), 7,46 (m, 2H), 7,30 (m, 5H), 4,70 (m, 1H), 4,20 (q, 2H), 2,90 (m, 2H), 2,60 (m, 2H), 1,0 (t, 3H)
SM : MH⁺ 478
HPLC: 100 %

### Exemple 12 : Acide (3S)-3-{[4-(4-Trifluorométhoxyphényl)thièn-2-yl]carboxamido)-4-phényl-butanoique

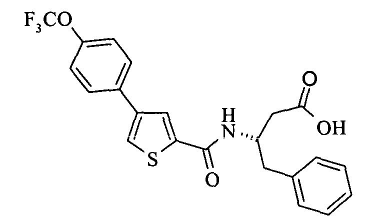

Le produit (104 mg) est obtenu selon le procédé de l'exemple 2 en utilisant comme substrat le composé obtenu dans l'exemple 11.
Rendement : 50 %
RMN ¹H (DMSO) δ (ppm) : 8,90 (m, 1H), 8,20 (m, 2H), 7,82 (d, 2H), 7,46 (m, 2H), 7,30 (m, 5H), 4,40 (m, 1H), 2,90 (m, 2H), 2,60 (m, 2H)
SM : MH- 448
HPLC : 100 %

### Exemple 13 : (4R)-3-{[4-(4-Trifluorométhoxyphényl)thièn-2-yl]carboxamido}-4-phényl-pentanoate de méthyle

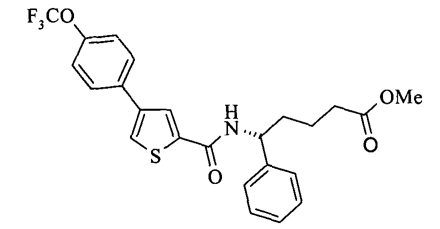

Le produit (21,3 mg) est obtenu selon le procédé de l'exemple 1 en utilisant comme substrat le produit de la préparation 4.
Rendement : 15 %
RMN ¹H (DMSO) δ (ppm) : 8,90 (m, 1H), 8,40 (s, 1H), 8,10 (s, 1H) ,7,82 (d, 2H), 7,46 (m, 1H), 7,40 (m, 6H), 7,20 (m, 1H), 4,90 (m, 1H), 3,50 (s, 3H), 2,40 (m, 2H), 2,20 (m, 2H)
SM : MH⁺ 464
HPLC : 100 %

### Exemple 14 : Acide (4R)-3-{[4-(4-Trifluorométhoxyphényl)thièn-2-yl]carboxamido}-4-phényl-pentanoique

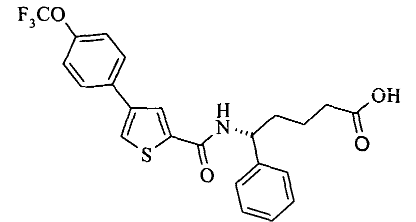

Le produit (173 mg) est obtenu selon le procédé de l'exemple 2 en utilisant comme substrat le composé obtenu dans l'exemple 13.
Rendement : 73 %
RMN ¹H (DMSO) δ (ppm) : 8,90 (m, 1H), 8,40 (s, 1H), 8,10 (s, 1H), 7,82 (d, 2H), 7,46 (m, 2H),7,36 (m, 5H), 4,90 (m, 1H), 2,40 (m, 2H), 2,20 (m, 2H)
SM : MH⁺ 450
HPLC : 98,9 %

### Exemple 15 : (2S)-{[4-(4-Cyclohexylphényl)thièn-2-yl]carboxamido}(phényl)acétate de méthyle

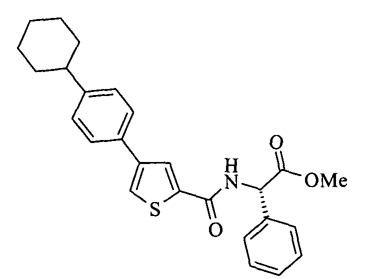

Le produit (0,155 g) est obtenu successivement selon le procédé du stade 1 de la préparation 1 en utilisant comme substrat le produit de la préparation 6 et de la préparation 5, puis le procédé de l'exemple 2 en utilisant comme substrat le produit obtenu au stade précédent, puis le procédé de l'exemple 1 en utilisant comme substrat le *(2S)*-amino(phényl)acétate d'éthyle.
Rendement : 55,4 %
RMN ¹H (DMSO) δ (ppm) : 9,20 (m, 1H), 8,40 (s, 1H), 8,10 (s, 1H) ,7,70 (d, 2H), 7,46 (m, 5H), 7,30 (m, 2H), 5,60 (d, 1H), 3,90 (s, 3H), 1,80 (m, 5H), 1,40 (m, 6H)
SM : MH⁺ 434
HPLC : 100 %

### Exemple 16 : Acide (2S)-{[4-(4-cyclohexylphényl)thièn-2-yl]carboxamido}(phényl) acétique

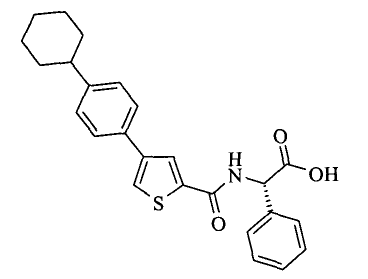

Le produit (37.4 mg) est obtenu selon le procédé de l'exemple 2 en utilisant comme substrat le composé obtenu dans l'exemple 15.
Rendement : 25 %
RMN ¹H (DMSO) δ (ppm) : 12,9 (m,1H), 9,10 (m, 1H), 8,40 (s, 1H), 8,10 (s, 1H) ,7,82 (d, 2H), 7,46 (m, 5H), 7,30 ( m, 2H), 5,50 (m,1H), 1,80 (m, 5H), 1,40 (m, 6H)
SM : MH⁺ 420
HPLC :100 %

### Exemple 17: (2S)-({4-[4-(4-Acétylphényl)phényl]thièn-2-yl}carboxamido) (phényl)acétate d'éthyle

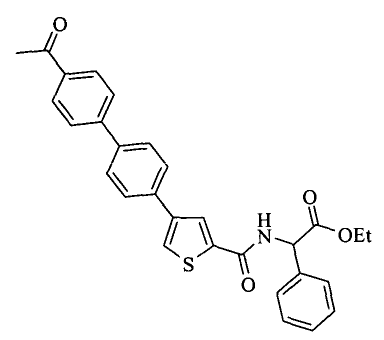

### Stade 1 : 4-[4-(4-Acétyl-phényl)phényl]-thiophène-2-carboxylate de méthyle

Le produit (1,73 g) est obtenu selon le procédé du stade 1 de la préparation 1 en utilisant comme substrats le produit de la préparation 6 et l'acide (4-acétyl-phényl)boronique.
Rendement : 76 %
SM : MH⁺ 337

### Stade 2 : Acide 4-[4-(4-acétyl-phényl)phényl]-thiophène-2-carboxylique

Le produit (1 g) est obtenu selon le procédé de l'exemple 2 en utilisant comme substrat le produit obtenu au stade 1 précédent.
Rendement : 61 %
SM : MH⁺ 323

### Stade 3 : (2S)-({4-[4-(4-Acétyl-phényl)phényl]thièn-2-yl}carboxamido) (phényl)acétate d'éthyle

Le produit (360 mg) est obtenu selon le procédé de l'exemple 1 en utilisant comme substrat le chlorhydrate de (2S)-amino(phényl)acétate d'éthyle.
Rendement : 44 %
RMN ¹H (CDCl₃) δ (ppm) : 9,20 (d, 1H), 8,60 (s,1H), 8,25 (s, 1H), 8,05 (d, 2H), 7,90 (d, 2H), 7,85 (s, 4H), 7,50 (m, 2H), 7,40 (m, 3H), 5,60 (d, 1H), 4,20 (m, 2H), 2,65 (s, 3H), 1,20 (t, 3H)

### Exemple 18 : Acide (2S)-4-[({4-[4-(4-Acétylphényl)phényl]thièn-2-yl}carbonyl)amino] (phényl)acétique

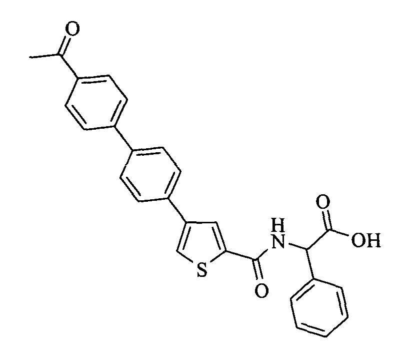

Le produit (16 mg) est obtenu selon le procédé de l'exemple 2 en utilisant comme substrat le composé obtenu dans l'exemple 17.
Rendement : 5 %
RMN ¹H (DMSO) δ (ppm) : 13,0 (bs, 1H), 9,10 (d, 1H), 8,60 (s, 1H), 8,25 (s, 1H), 8,05 (d, 2H), 7,90 (m, 6H), 7,50 (m, 2H), 7,40 (m, 3H), 5,55 (d, 1H), 2,60 (s, 3H)
SM : MH⁺ 456
HPLC : 95,9 %

### Exemple 19 : (2R)-3-({4-[4-(4-Acétyl-phényl)phényl]thièn-2-yl}carboxamido) (phényl)propanoate d'éthyle

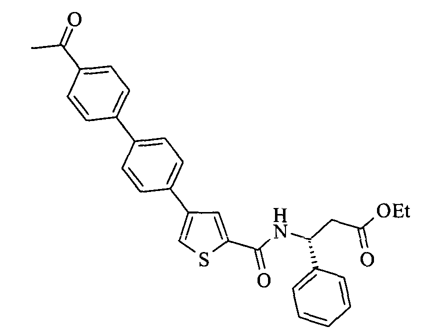

Le produit (1,012 g) est obtenu selon le procédé de l'exemple 17 en remplaçant le chlorhydrate de (*2S*)-amino(phényl)acétate d'éthyle par le chlorhydrate de (*2R*)-amino(phényl)propanoate d'éthyle.
Rendement : 66 %
RMN ¹H (DMSO) δ (ppm) : 9,10 (d, 1H), 8,30 (s, 1H), 8,25 (s, 1H), 8,15 (d, 2H), 7,90 (m, 6H), 7,35 (m, 5H), 5,40 (m, 1H), 4,10 (q, 2H), 3,0 (m, 2H), 2,60 (s, 3H), 1,30 (t, 3H)

### Exemple 20 : Acide (2R)-3-({4-[4-(4-Acétyl-phényl)phényl]thièn-2-yl}carboxamido) (phényl)propanoique

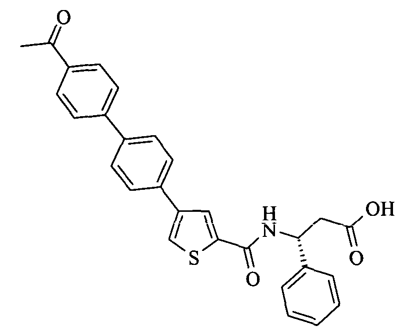

Le produit (0,641 g) est obtenu selon le procédé de l'exemple 2 en utilisant comme substrat le composé obtenu dans l'exemple 19.
Rendement : 91 %
RMN ¹H(DMSO) δ (ppm) : 9,10 (d, 1H), 8,3(s, 1H), 8,25 (s, 1H), 8,15 (d, 2H), 7,90 (m, 6H), 7,35 (m, 5H), 5,40 (m, 1H), 3,0 (m, 2H), 2,60 (s, 3H)
SM : MH⁺ 470
HPLC: 100 %

### Exemple 21 : (2R)-3-({4-[4-(Pyridin-4-yl)phényl]-thièn-2-yl}carboxamido) (phényl)propanoate d'éthyle

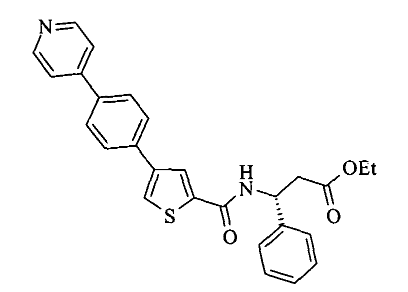

### Stade 1 : Chlorydrate de 4-[4-(Pyridin-4-yl)phényllthiophène-2-carboxylate de méthyle

Le produit brut (60 g) est obtenu sous forme de solide jaune en appliquant le procédé du stade 1 de la préparation 1 en utilisant comme substrats le produit de la préparation 6 et l'acide (4-pyridyl)boronique. La phase organique obtenue après extraction est acidifiée avec une solution d'acide chlorhydrique 2M afin de précipiter le produit désiré, enfin isolé par filtration.
RMN ¹H (DMSO) δ (ppm) : 8,91 (d, 2H), 8,50 (s, 1H), 8,38 (s, 1H), 8,36 (d, 2H), 8,10 (d, 2H), 8,06 (d, 2H), 3.45 (s, 3H)

### Stade 2 : Chlorhydrate d'acide 4-[4-(4-acétylphényl)phényl]thiophène-2-carboxylique

Le produit (19.4 g) est obtenu selon le procédé de l'exemple 2 en utilisant comme substrat le produit obtenu au stade 1 précédent.
Rendement pour les stades 1 et 2: 44.5 %
RMN ¹H (DMSO) δ (ppm) : 8,88 (d, 2H), 8,43 (s, 1H), 8,30 (d, 2H), 8,28 (s, 1H), 8,08 (d, 2H), 8,02 (d, 2H)

### Stade 3 : (2R)-3[([4-[4-(4-A cétylphényl)phényllthièn-2-yl]carboxamido) (phényl)propanoate d'éthyle

Le produit (1,43 g) est obtenu selon le procédé de l'exemple 1 en utilisant comme substrat le chlorhydrate de (*2R*)-amino(phényl)propanoate d'éthyle.
Rendement : 100 %
RMN¹H (DMSO) δ (ppm) : 8,15 (d, 1H), 8,76 (d, 2H), 8,35 (s, 1H), 8,22 (s, 1H), 7,95 (d, 2H), 7,90 (d, 2H), 7,88 (d, 2H), 7,44 (d, 2H), 7,35 (t, 2H), 7,26 (t, 1H), 5,42 (q, 1H), 4,05 (q, 2H), 2,95 (m, 2H), 1,10 (t, 3H)
HPLC: 100 %

### Exemple 22 : Chlorhydrate d'acide (2R)-3-({[4-[(pyridin-4-yl)phényl]-thièn-2-yl}carboxamido)(phényl)propanoïque

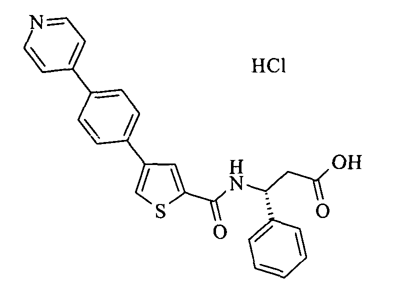

Le produit (0,990 g) est obtenu selon le procédé de l'exemple 2 en utilisant comme substrat le composé obtenu dans l'exemple 21.
Rendement : 68 %
RMN ¹H (DMSO) δ (ppm) : 9,02 (d, 1H), 8,88 (d, 2H), 8,46 (s, 1H), 8,34 (d, 2H), 8,30 (s, 1H), 8,11 (d, 2H), 7,95 (d, 2H), 7,44 (d, 2H), 7,35 (t, 2H), 7,26 (t, 1H), 5,43 (q, 1H), 2,89 (m, 2H)
HPLC : 100 %

### Exemple 23 : N-(3-Benzyloxy-1-phényl-2-oxopropyl)-4-(4-méthoxyphényl)thiophène-2-carboxamide

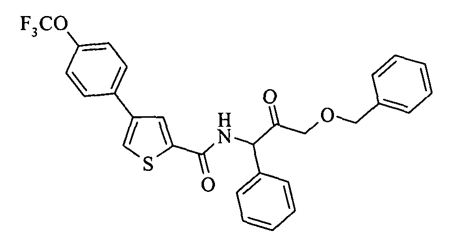

Le produit (17,8mg) est obtenu selon le procédé de l'exemple 1 en utilisant comme substrat le composé obtenu dans la préparation 7.
Rendement : 23 %
RMN ¹H (DMSO) δ (ppm) : 9,10 (d, 1H), 8,50 (s, 1H), 8,25 (s, 1H), 7,80 (d, 2H), 7,40 (m, 12H), 5,90 (m, 1H), 4,40 (m, 4H)
SM : MH⁺ 526
HPLC : 100 %

### Exemple 24 : Etudes pharmacologiques des composés de l'invention:

### Evaluation in vitro de l'activité inhibitrice des composés de l'invention sur la MMP-12 :

L'activité inhibitrice des composés de formule (I) sur la métalloprotéinase-12 est évaluée en testant la capacité des composés de l'invention à inhiber la protéolyse d'un peptide substrat de la MMP-12.
Le peptide substrat utilisé (peptide-1 fluorigénique : FP-1) dans le test présente la séquence suivante : Mca-Pro-Leu-Gly-Leu-Dap(Dnp)-Ala-Arg-NH₂
L'activité inhibitrice d'un composé de formule (I) est exprimée en valeur d'IC₅₀, qui représente la concentration en inhibiteur pour laquelle un taux d'inhibition de 50% de la métalloprotéinase est observé.
La réaction commence par l'addition séquentielle de 41 µl de substrat FP-1 (concentration finale de 10 µM) à une solution tampon de 50 mM de Tris-HCl et 10 mM de CaCl₂, et contenant 5 mM d'acide hydroxamique et 5 µl de l'enzyme diluée dans une solution tampon 0,005% Brij-35. Les microplaques sont incubées pendant 20 minutes à température ambiante. Les composés de l'invention sont testés à des concentrations variants de 0,3 à 30 µM.
La mesure de la quantité de protéolyse du substrat peptidique est suivie par une mesure d'absorbance à 405 nm en utilisant un spectrophotomètre de microplaques, à la température ambiante. Les valeurs d'IC₅₀ sont calculées à partir de courbes dans lesquelles le pourcentage de l'activité catalytique relative au contrôle est représenté sur l'axe des X et la concentration en inhibiteur est représentée sur l'axe des Y.

Le test décrit ci-dessus pour l'inhibition de la MMP-12 est adapté et utilisé pour déterminer la capacité des composés de formule (I) à inhiber les métalloprotéinases MMP-1, MMP-2, MMP-3, MMP-7, MMP-9, MMP-13 et MMP-14. Les résultats obtenus montrent que les composés de l'invention ont généralement des valeurs d'IC₅₀ pour la MMP-12 qui sont de 5 à plus de 100 fois plus faibles que les valeurs d'IC₅₀ obtenues pour le même composé avec les autres métalloprotéinases testées, prouvant ainsi leur capacité d'inhibition sélective vis-à-vis de la métalloprotéinase-12 (MMP-12). Plus spécifiquement les composés de la présente invention montrent généralement une sélectivité avec un facteur supérieur à 50 vis-à-vis des métalloprotéinases mentionnées précédemment, excepté à l'égard de la MMP-13. Ainsi, les composés de la présente invention montrent également une activité inhibitrice sur la MMP-13 permettant également d'utiliser les compositions pharmaceutiques contenenant un ou plusieurs composés de l'invention pour le traitement des pathologies liées à une activité de la MMP-13. Parmi ces pathologies, on peut citer à titre indicatif et non limitatif, le cancer, l'ostéoporose, l'ostéoarthrite, l'arthrite, l'arthrite rhumatoïde, l'athéorosclérose, la sclérose en plaques, l'insuffisance cardiaque, l'asthme et les broncho-pneumopathies chroniques obstructives.

A titre d'exemple et sans limitation de l'invention, le tableau présente quelques résultats d'activité des composés de l'invention vis-à-vis de la MMP-12 et de la MMP-13.

| Exemple | IC₅₀ (µM) MMP-12 | IC₅₀ (µM) MMP-13 |
|---|---|---|
| 2 | 0,38 | 4,5 |
| 4 | 0,47 | 5,6 |
| 6 | 0,420 | 3,6 |
| 8 | 0,180 | 2,2 |
| 9 | 0,077 | 0,96 |
| 12 | 0,530 | 8,3 |
| 16 | 0,540 | 6,5 |
| 18 | 0,040 | 0,120 |
| 20 | 0,028 | 0,069 |
| 22 | 0,014 | 0,270 |

## Revendications

1. Composés de formule (I) : 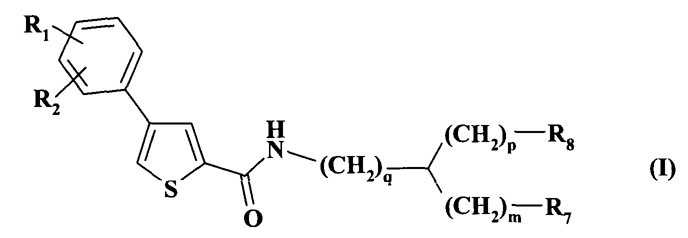 dans laquelle:
• R₁ et R₂, identiques ou différents, indépendamment l'un de l'autre, représentent chacun un groupement choisi parmi :
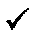 hydrogène, halogène, cyano, nitro, halogéno(C₁-C₆)alkyle, halogéno(C₁-C₆)alkoxy, (C₁-C₆)alkyle, (C₂-C₆)alkènyle, (C₂-C₆)alkynyle,
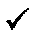 -OR₄, -NR₄R₅, -S(O)ₙR₄, -C(O)R₄, -CO₂R₄, -O-C(O)R₄, -C(O)NR₄R₅, -NR₅-C(O)R₄, - NR₅-SO₂R₄, -T-CN, -T-OR₄, -T-OCF₃, -T-NR₄R₅, -T-S(O)ₙR₄, -T-C(O)R₄, - T-CO₂R₄, -T-O-C(O)R₄, -T-C(O)NR₄R₅, -T-NR₅-C(O)R₄, -T-NR₅-SO₂R₄, -R₆, et - T-R₆ dans lesquels :
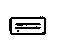 n représente un entier compris entre 0 et 2 inclus,
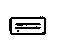 T représente une chaîne (C₁-C₆)alkylène linéaire ou ramifiée, éventuellement substituée par un groupement choisi parmi oxo, halogène, (C₁-C₆)alkoxy, hydroxy, amino, mono(C₁-C₆)alkylamino et di(C₁-C₆)alkylamino, et/ou dans laquelle éventuellement un des atomes de carbone est remplacé par un atome d'oxygène, un atome de soufre, un groupement -NH-, ou un groupement - N(C₁-C₆)alkyle-, (étant entendu que dans le cas où un des atomes de carbone est remplacé par un groupement tel que défini précédemment alors ladite chaîne alkylène comporte au moins un enchaînement de deux atomes)
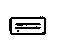 R₄ représente un atome d'hydrogène, un groupement (C₁-C₆)alkyle, aryle, cycloalkyle, ou un hétérocycle,
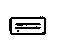 R₅ représente un atome d'hydrogène ou un groupement (C₁-C₆)alkyle,
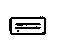 R₆ représente un groupement choisi parmi aryle, cycloalkyle, et un hétérocyle, chacun de ces groupements étant éventuellement substitués par un à cinq groupements, identiques ou différents, indépendamment l'un de l'autre choisis parmi halogène, cyano, nitro, oxo, halogéno(C₁-C₆)alkyle, halogéno(C₁-C₆)alkoxy, (C₁-C₆)alkyle, (C₁-C₆)alkènyle, -OR₄₀, -NR₄₀R₅₀, -S(O)ₙ₁R₄₀, -C(O)R₄₀, -CO₂R₄₀, -O-C(O)R₄₀, -C(O)NR₄₀R₅₀, -NR₅₀-C(O)R₄₀, -NR₅₀-SO₂R₄₀, -T₁-CN, -T₁-OR₄₀, -T₁-OCF₃, -T₁-NR₄₀R₅₀, -T₁-S(O)ₙR₄₀, -T₁-C(O)R₄₀, -T₁-CO₂R₄₀, -T₁-O-C(O)R₄₀, -T₁-C(O)NR₄₀R₅₀, -T₁-NR₅₀-C(O)R₄₀, et - T₁-NR₅₀-SO₂R₄₀ dans lesquels R₄₀, R₅₀, T₁ et n₁ ont respectivement les mêmes significations que R₄, R₅, T et n tels que définis précédemment ;
• m représente un entier compris entre 0 et 4 inclus ;
• p représente un entier compris entre 0 et 4 inclus ;
• q représente un entier compris entre 0 et 4 inclus ;
• R₇ représente un groupement choisi parmi aryle, cycloalkyle et un hétérocycle chacun de ces groupements étant éventuellement substitué par un à trois groupements, identiques ou différents, indépendamment l'un de l'autre choisis parmi parmi halogène, cyano, nitro, halogéno(C₁-C₆)alkyle, halogéno(C₁-C₆)alkoxy, (C₁-C₆)alkyle, hydroxy, (C₁-C₆)alkoxy, amino, mono(C₁-C₆)alkylamino, di(C₁-C₆)alkylamino, mercapto, (C₁-C₆)alkylthio, (C₁-C₇)acyle, (C₁-C₆)alkylsulfoxyde, carboxy, et (C₁-C₆)alkoxycarbonyl ;
• R₈ représente un groupement choisi parmi carboxy, (C₁-C₆)alkoxycarbonyl, (C₁-C₇)acyle, hydroxy(C₁-C₆)alkylcarbonyl, (C₁-C₆)alkoxy(C₁-C₆)alkylcarbonyl, tetrazole, amino, aminocarbonyl, et amino(C₁-C₆)alkylcarbonyl, (la partie amino dans chacun des groupements portant cette fonction étant éventuellement substituée par un ou deux groupements, identiques ou différents, indépendamment l'un de l'autre choisis parmi (C₁-C₆)alkyl et cycloalkyle) ;
leurs isomères, ainsi que leurs sels d'addition à un acide ou à une base pharmaceutiquement acceptable,
étant entendu que :
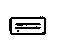 par groupement aryle on comprend un système monocyclique ou bicyclique aromatique comprenant de 4 à 10 atomes de carbone, étant compris que dans le cas d'un système bicyclique l'un des cycles présente un caractère aromatique et l'autre cycle est aromatique ou insaturé,
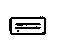 par groupement cycloalkyle on comprend un système monocyclique ou bicyclique fusionné ou ponté, saturé ou partiellement insaturé, comprenant de 3 à 12 atomes de carbone,
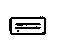 par un groupement hétérocycle on comprend un système monocyclique ou bicyclique fusionné ou ponté, de 3 à 12 chaînons, saturé, insaturé ou aromatique, comprenant de 1 à 4 hétéroatomes, identiques ou différents, choisis parmi oxygène, soufre et azote, et contenant éventuellement 1 ou 2 groupements oxo ou thioxo, étant compris que dans le cas d'un système bicyclique l'un des cycles présente un caractère aromatique et l'autre cycle est aromatique ou insaturé, ou les deux cycles sont saturés, ou l'un des cyles est saturé et l'autre cycle est insaturé, ou les deux cycles sont insaturés ;
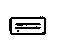 par groupement (C₁-C₆)alkyle on comprend une chaîne carbonée linéaire ou ramifiée comprenant de 1 à 6 atomes de carbone,
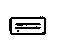 par groupement halogéno(C₁-C₆)alkyle on comprend une chaîne carbonée linéaire ou ramifiée comprenant de 1 à 6 atomes de carbone et substituée par 1 à 6 atomes d'halogène,
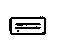 par groupement halogéno(C₁-C₆)alkoxy on comprend une chaîne carbonée linéaire ou ramifiée comprenant de 1 à 6 atomes de carbone et substituée par 1 à 6 atomes d'halogène, ladite chaîne étant reliée au composé de formule (I) par un atome d'oxygène,
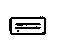 par atome d'halogène on comprend un atome choisi parmi brome, chlore, fluor et iode,
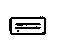 par groupement acyle on comprend un atome d'hydrogène, un groupement alkyle tel que défini précédemment, un cycloalkyle de 3 à 6 atomes de carbone, ou un groupement phényle lié au travers d'un groupement oxo aux composés de formule (I).

2. Composés de formule (I) selon la revendication 1 **caractérisés en ce que**:
• R₁ représente un groupement choisi parmi halogéno(C₁-C₆)alkoxy, (C₁-C₆)alkyle, - OR₄, -SR₄, et -R₆ dans lesquel :
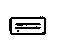 R₄ représente un atome d'hydrogène ou un groupement (C₁-C₆)alkyl,
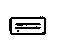 R₆ représente un groupement choisi parmi phényle, cyclohexyle, et un hétérocycle, chacun de ces groupements étant éventuellement substitués par un à deux groupements, identiques ou différents, indépendamment l'un de l'autre choisis parmi halogène, halogéno(C₁-C₆)alkyl-, halogéno(C₁-C₆)alkoxy-, (C₁-C₆)alkyle, cyano, -T₁-CN, -OR₄₀, -T₁-OR₄₀, -NR₄₀R₅₀, -S(O)ₙ₁R₄₀, et - C(O)R₄₀, dans lesquels :
- R₄₀ représente un groupement choi parmi atome d'hydrogène, (C₁-C₆)alkyl et phényle,
- R₅₀ représente un groupement choisi parmi atome d'hydrogène et (C₁-C₆)alkyl,
- T₁ représente une chaîne (C₁-C₆)alkylène linéaire ou ramifiée,
- n1 représente un entier compris entre 0 et 2 inclus ;
• R₂ représente un atome d'hydrogène ;
• m représente un entier compris entre 0 et 4 inclus ;
• p représente un entier compris entre 0 et 4 inclus ;
• q représente un entier compris entre 0 et 4 inclus ;
• R₇ représente un groupement choisi parmi phényle, cycloalkyle monocyclique et un hétérocycle monocyclique à 5 ou 6 chaînons et comprenant un ou deux hétéroatomes, identiques ou différents, choisis parmi oxygène et azote, chacun de ces groupements étant éventuellement substitué par un à deux groupements, identiques ou différents, indépendamment l'un de l'autre choisis parmi parmi halogène, cyano, nitro, halogéno(C₁-C₆)alkyle, halogéno(C₁-C₆)alkoxy, (C₁-C₆)alkyle, hydroxy, (C₁-C₆)alkoxy, amino, mono(C₁-C₆)alkylamino, di(C₁-C₆)alkylamino, mercapto, (C₁-C₆)alkylthio, (C₁-C₇)acyle, (C₁-C₆)alkylsulfoxyde, carboxy, et (C₁-C₆)alkoxycarbonyl ;
• R₈ représente un groupement choisi parmi carboxy, (C₁-C₆)alkoxycarbonyl, aminocarbonyl, (C₁-C₆)alkylcarbonyl, amino, hydroxy(C₁-C₆)alkylcarbonyl, (C₁-C₆)alkoxy(C₁-C₆)alkylcarbonyl, amino(C₁-C₆)alkylcarbonyl, (la partie amino dans chacun des groupements portant cette fonction étant éventuellement substituée par un ou deux groupements, identiques ou différents, indépendamment l'un de l'autre (C₁-C₆)alkyl), leurs isomères, ainsi que leurs sels d'addition à un acide ou à une base pharmaceutiquement acceptable,

3. Composés de formule (I) selon l'une quelconque des revendications 1 ou 2 **caractérisés en ce que** le groupement phényle des composés de formule (I) est substitué par un groupement R₁ tel que défini dans la formule (I) situé en position *para,* leurs isomères, ainsi que leurs sels d'addition à un acide ou à une base pharmaceutiquement acceptable.

4. Composés de formule (I) selon les revendications 1 à 3 **caractérisés en ce que** R₁ représente un groupement choisi parmi trifluorométhoxy, 4-acétylphényle, 4-pyridyle, 3-pyridyle, N-pyrrolydinyle, 1-méthylpyrrol-3-yl, 3,6-dihydro-2H-pyridin-1-yl, cyclohexyle, 2-hydroxy-4-pyridyle, et 2-hydroxyphényle, leurs isomères, ainsi que leurs sels d'addition à un acide ou à une base pharmaceutiquement acceptable.

5. Composés de formule (I) selon l'une quelconque des revendications 1 à 3 **caractérisés en ce que** R₁ représente un groupement choisi parmi trifluorométhoxy, 4-acétylphényle, cyclohexyle et 4-pyridyle, leurs isomères, ainsi que leurs sels d'addition à un acide ou à une base pharmaceutiquement acceptable.

6. Composés de formule (I) selon l'une quelconque des revendications 1 ou 2 **caractérisés en ce que** q est égal à zéro, leurs isomères, ainsi que leurs sels d'addition à un acide ou à une base pharmaceutiquement acceptable.

7. Composés de formule (I) selon l'une quelconque des revendications 1 ou 2 **caractérisés en ce que** m représente un entier choisi parmi zéro et un, leurs isomères, ainsi que leurs sels d'addition à un acide ou à une base pharmaceutiquement acceptable.

8. Composés de formule (I) selon l'une quelconque des revendications 1 ou 2 **caractérisés en ce que** p représente un entier choisi parmi zéro et un, leurs isomères, ainsi que leurs sels d'addition à un acide ou à une base pharmaceutiquement acceptable.

9. Composés de formule (I) selon l'une quelconque des revendications 1 ou 2 **caractérisés en ce que** R₇ représente un groupement phényle, leurs isomères, et leurs sels d'addition à un acide ou à une base pharmaceutiquement acceptable.

10. Composés de formule (I) selon l'une quelconque des revendications 1 ou 2 **caractérisés en ce que** R₈ représente un groupement choisi parmi carboxy et aminocarbonyle, leurs isomères, ainsi que leurs sels d'addition à un acide ou à une base pharmaceutiquement acceptable.

11. Composés de formule (I) selon la revendication 1 qui sont 1' :
- (2S)- {[4-(4-Trifluorométhoxyphényl)thièn-2-yl]carboxamido}(phényl)acétate d'éthyle
- Acide (2S)-({[4-(4-trifluorométhoxyphényl)thièn-2-yl]carboxamido}(phényl) acétique
- (2R)- {[4-(4-Trifluorométhoxyphényl)thièn-2-yl]carboxamido}(phényl)acétate d'éthyle
- Acide (2R)-{[4-(4-trifluorométhoxyphényl)thièn-2-yl]carboxamido}(phényl) acétique
- 3-{[4-(4-Trifluorométhoxyphényl)thièn-2-yl]carboxamido)-3-phényl-propanoate d'éthyle
- Acide {[4-(4-Trifluorométhoxyphényl)thièn-2-yl]carboxamido}(phényl) propanoique
- Acide (2S)-{[4-(4-Trifluorométhoxyphényl)thièn-2-yl]carboxamido} (phényl) propanoique
- Acide (2R)-{[4-(4-Trifluorométhoxyphényl)thièn-2-yl]carboxamido} (phényl) propanoique
- N-(3-amino-3-oxo-1-phénylpropyl)-4-(4-trifluorométhoxyphényl)thiophène-2-carboxamide
- 4-(4-Trifluorométhoxyphényl)-N-[(2S)-2-(cyclohexylamino)-2-phényléthanoyl] thiophène-2-carboxamide
- (3S)-3-{[4-(4-Trifluorométhoxyphényl)thièn-2-yl]carboxamido}-4-phénylbutanoate d'éthyle
- Acide (3S)-3-{[4-(4-Trifluorométhoxyphényl)thièn-2-yl]carboxamido)-4-phényl-butanoique
- (4R)-3-{[4-(4-Trifluorométhoxyphényl)thièn-2-yl]carboxamido}-4-phényl-pentanoate de méthyle
- Acide (4R)-3-{[4-(4-Trifluorométhoxyphényl)thièn-2-yl]carboxamido} - 4-phényl-pentanoique
- (2S)-{[4-(4-Cyclohexylphényl)thièn-2-yl]carboxamido}(phényl)acétate de méthyle
- Acide (2S)-{[4-(4-cyclohexylphényl)thièn-2-yl]carboxamido}(phényl) acétique
- (2S)-({4-[4-(4-Acétylphényl)phényl]thièn-2-yl}carboxamido) (phényl)acétate d'éthyle
- Acide (2S)-4-[({4-[4-(4-Acétylphényl)phényl]thièn-2-yl}carbonyl) amino] (phényl)acétique
- (2R)-3-({4-(4-(4-Acétyl-phényl)phényl]thièn-2-yl}carboxamido)(phényl)propanoate d'éthyle
- Acide (2R)-3-({4-[4-(4-Acétyl-phényl)phényl]thièn-2-yl}carboxamido)(phényl) propanoique
- (2R)-3-({4-[4-(Pyridin-4-yl)phényl]-thièn-2-yl}carboxamido)(phényl)propanoate d'éthyle
- Chlorhydrate d'acide (2R)-3-({[4-[(pyridin-4-yl)phényl]-thièn-2-yl}carboxamido) (phényl)propanoïque
- et le N-(3-Benzyloxy-1-phényl-2-oxopropyl)-4-(4-méthoxyphényl)thiophène-2-carboxamide,
leurs isomères ainsi que leurs sels d'addition à un acide ou à une base pharmaceutiquement acceptable.

12. Procédé de préparation des composés de formule (I) **caractérisé en ce qu'**on utilise comme produit de départ un composé de formule (II) : 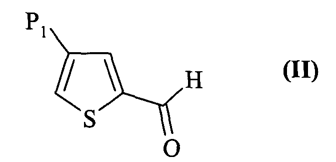 dans laquelle P₁ représente un atome d'halogène ou un groupement triflate, composés de formule (II) qui sont soumis à des conditions d'oxydation en présence, par exemple, de nitrate d'argent en milieu basique et polaire, pour donner les composés de formule (III) : 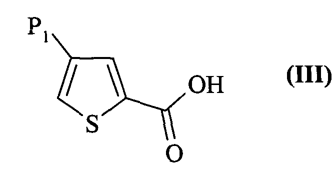 dans laquelle P₁ est tel que défini précédemment,
composés de formule (III) qui sont éventuellement transformés en chlorures d'acide correspondant (IV) par action du chlorure d'oxalyle par exemple, 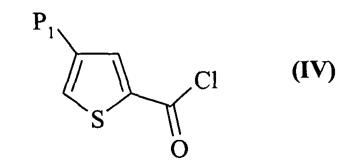 dans laquelle P₁ est tel que défini précédemment,
ou qui sont traités directement, dans des conditions de couplage peptidique en présence d'un agent de couplage et dans un milieu basique, avec un composé de formule (V) : 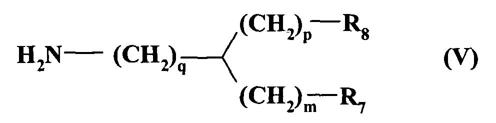 dans laquelle R₇, R₈, m, p et q ont les mêmes significations que dans les composés de formule (I),
pour conduire aux composés de (VI) : 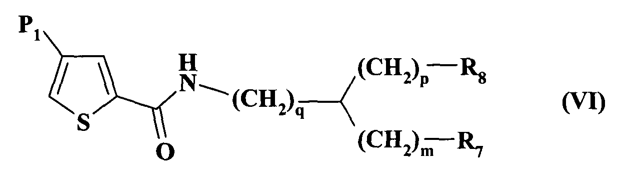 dans laquelle P₁, R₇, R₈, m, p et q sont tels que définis précédemment,
composés de formule (VI) qui sont :
• soit mis à réagir, dans des conditions basiques de couplage au palladium, avec un composé de formule (VII) : 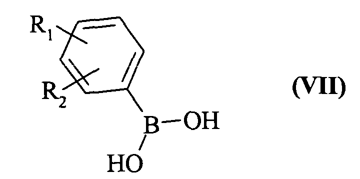 dans laquelle R₁ et R₂ ont les mêmes significations que dans les composés de formule (I), pour conduire aux composés de formule (I) ;
• soit traités avec de l'hexaméthyldiétain, en présence d'un catalyseur au palladium, pour conduire aux composés de formule (VIII) : 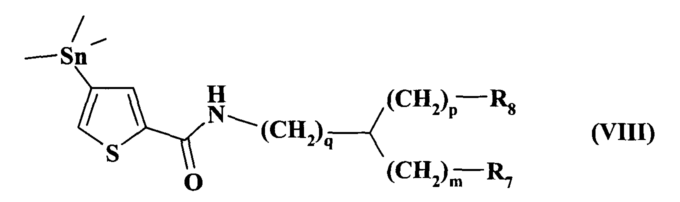 dans laquelle R₇, R₈, m, p et q sont tels que définis précédemment,
composés de formule (VIII) qui sont mis à réagir :
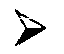 avec un composé de formule (IX) : 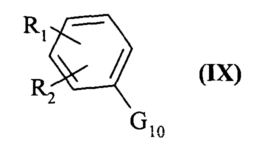 dans laquelle R₁ et R₂ ont les mêmes significations que dans la formule (I) et G₁₀ représentent un atome d'halogène choisi parmi chlore et brome ou un groupement triflate,
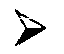 soit en présence de triphénylphosphine arsenic et d'un catalyseur au palladium, dans le cas où G₁₀ représente un groupement triflate,
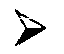 soit en présence d'un composé halogénocuivrique tel que CuBr₂ et d'un catalyseur au palladium, dans des conditions de solvant polaire, dans le cas où G₁₀ représente un atome d'halogène,
pour conduire également aux composés de formule (I) ;
• soit traités par du bis(pinacolato)di-borane suivi par une réaction d'oxydation pour conduire aux composés de formule (VIa) : 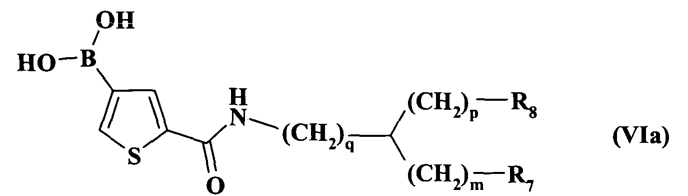 dans laquelle P₁, R₇, R₈, m, p et q sont tels que définis précédemment,
composés de formule (VIa) qui sont mis à réagir dans des conditions basiques de couplage au palladium avec un composé de formule (IX) : 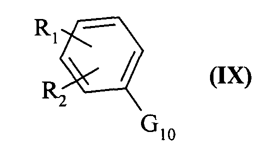 dans laquelle R₁, R₂ et G₁₀ sont tels que définis précédemment,
pour conduire également aux composés de formule (I) ;
• soit mis à réagir avec un composé de formule (IXa): 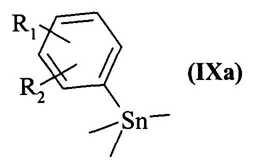 dans laquelle R₁, et R₂ sont tels que définis dans la formule (I),
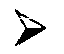 soit en présence de triphénylphosphine arsenic et d'un catalyseur au palladium, dans le cas où P₁ dans les composés de formule (VI) représente un groupement triflate,
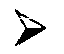 soit en présence d'un composé halogénocuivrique tel que CuBr₂ et d'un catalyseur au palladium, dans des conditions de solvant polaire, dans le cas où P₁ dans les composés de formule (VI) représente un atome d'halogène,
pour conduire également aux composés de formule (1) ;
composés de formule (I) dans le cas particulier où ils représentent des composés de formule (I/a) dans lesquels R₂ représente un atome d'hydrogène, R₁ représente un groupement hydroxy ou un atome d'halogène (Hal) et R₇, R₈, m, p et q ont les mêmes significations que dans la formule (1) : 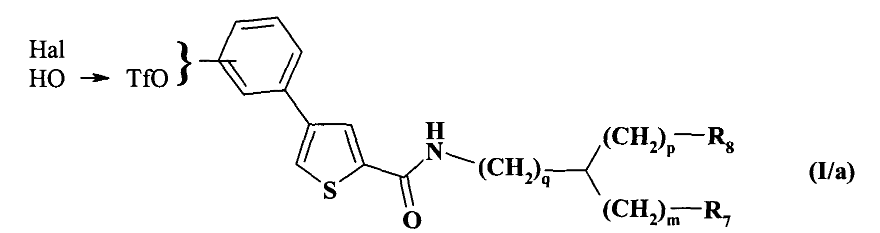 composés de formule (I/a) qui peuvent alors être traités préalablement par de l'anhydride trifluorométhanesulfonique en présence d'une base forte, dans le cas où R₁ représente un groupement hydroxy, pour obtenir le dérivé activé triflate,
lesdits composés portant un groupement R₁ prenant la définition halogène ou triflate pouvant alors
o soit être mis à réagir en condition basique et en présence d'un catalyseur au palladium avec un composé de formule (X) : 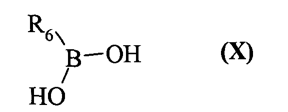 dans laquelle R₆ est tel que défini dans la formule (I), c'est à dire qu'il représente un groupement choisi parmi aryle, cycloalkyle, et un hétérocycle,
pour conduire aux composés de formule (I/b), cas particulier des composés de formule (I) : 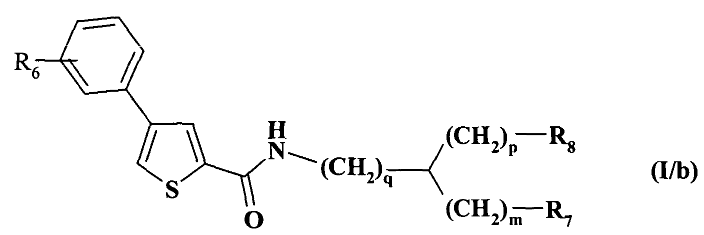 dans laquelle R₆, R₇, R₈, m, p et q sont tels que définis précédemment,
o soit être traités par du bis(pinacolato)di-borane suivi par d'une réaction d'oxydation, pour conduire aux composés de formule (XI) : 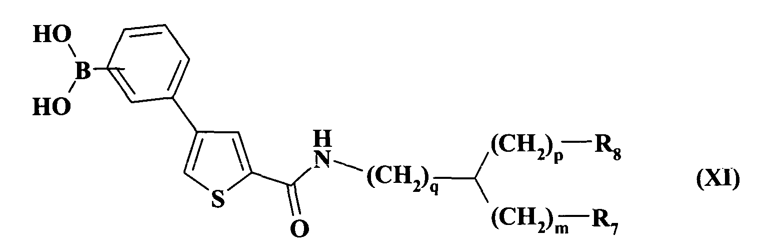 dans laquelle R₇, R₈, m, p et q sont tels que définis précédemment,
composés de formule (XI) qui sont mis à réagir en condition basique et en présence d'un catalyseur au palladium avec un composé de formule (Xa) :
R₆-P₂ **(Xa)**
dans laquelle R₆ est tel que défini dans la formule (I), c'est à dire qu'il représente un groupement choisi parmi aryle, cycloalkyle, et un hétérocycle, et P₂ représente un atome d'halogène ou un groupement triflate,
pour conduire également aux composés de formule (I/b) ;
o soit être traités avec de l'hexaméthyldiétain, en présence d'un catalyseur au palladium, pour conduire aux composés de formule (XIa) : 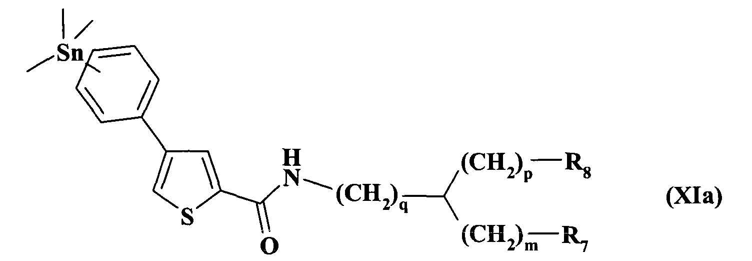 dans laquelle R₇, R₈, m, p et q sont tels que définis précédemment,
composés de formule (XIa) qui sont mis à réagir avec un composé de formule (Xa) tel que défini précédemment :
R₆-P₂ **(Xa)**
dans laquelle R₆ est tel que défini dans la formule (I), P₂ représente un atome d'halogène ou un groupement triflate,
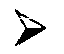 soit en présence de triphénylphosphine arsenic et d'un catalyseur au palladium, dans le cas où P₂ représente un groupement triflate,
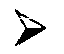 soit en présence d'un composé halogénocuivrique tel que CuBr₂ et d'un catalyseur au palladium, dans des conditions de solvant polaire, dans le cas où P₂ représente un atome d'halogène,
pour conduire également aux composés de formule (I/b) ;
o soit être mis à réagir avec un composé de formule (Xb):
R₆-SnMe₃ **(Xb)**
dans laquelle R₆ est tel que défini précédemment,
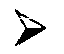 soit en présence de triphénylphosphine arsenic et d'un catalyseur au palladium, dans le cas où les composés de formule (I/a) comportent un groupement triflate,
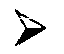 soit en présence d'un composé halogénocuivrique tel que CuBr₂ et d'un catalyseur au palladium, dans des conditions de solvant polaire, dans le cas où les composés de formule (I/a) comportent un atome d'halogène,
pour conduire également aux composés de formule (I/b) ;
o soit être mis à réagir dans des conditions de couplage au palladium, en milieu basique, avec un composé de formule (XII) :
R₆'-H **(XII)**
dans laquelle R₆' représente un hétérocycle azoté éventuellement substitué par un ou plusieurs groupements tels qu'ils sont définis pour les substituants du groupement R₆ au sein des composés de formule (I),
pour conduire aux composés de formule (I/c), cas particulier des composés de formule (I) : 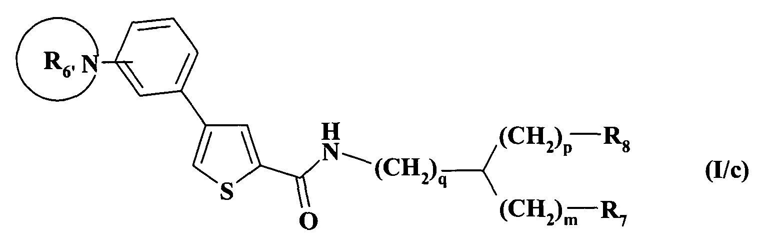 dans laquelle R₇, R₈, m, p et q sont tels que définis précédemment, et R₆, représente un hétérocycle azoté éventuellement substitué tel que défini dans la formule (I),
les composés (I/a) à (I/c) formant ensemble des composés de l'invention, qui sont purifiés, le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

13. Procédé de préparation des composés de formule (I), **caractérisé en ce qu'**on utilise comme produit de départ un composé de formule (II) : 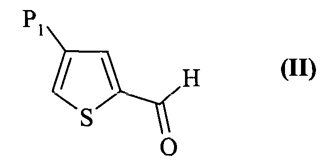 dans laquelle P₁ représente un atome d'halogène ou un groupement triflate,
composés de formule (II) qui sont soumis à des conditions d'oxydation en présence, par exemple, de nitrate d'argent en milieu basique et polaire, pour donner les composés de formule (III) : 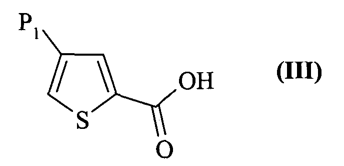 dans laquelle P₁ est tel que défini précédemment,
composés de formule (III) dont la fonction acide est estérifiée par action d'un alcool en présence d'un acide fort, pour conduire aux composés de formule (XX) : 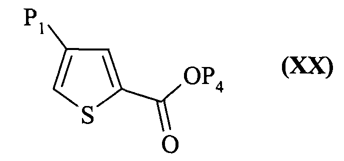 dans laquelle P₁ est tel que défini précédemment, et P₄ représente un groupement (C₁-C₄)alkyle linéaire ou ramifié,
composés de formule (XX) qui sont :
• soit mis à réagir, dans des conditions basiques de couplage au palladium, avec un composé de formule (VII) : 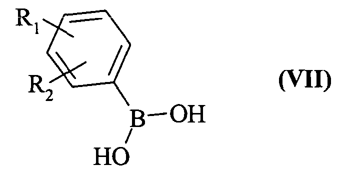 dans laquelle R₁ et R₂ ont les mêmes significations que dans les composés de formule (I),
pour conduire aux composés de formule (XXa) : 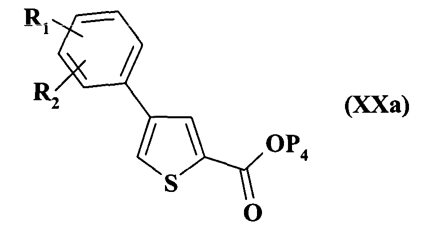 dans laquelle R₁, R₂ et P₄ sont tels que définis précédemment,
• soit traités avec de l'hexaméthyldiétain, en présence d'un catalyseur au palladium, pour conduire aux composés de formule (XXI) : 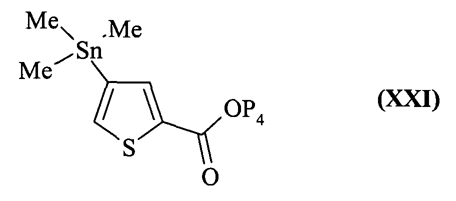 dans laquelle P₄ est tel que défini précédemment,
composés de formule (XXI) qui sont mis à réagir :
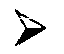 avec un composé de formule (IX) : 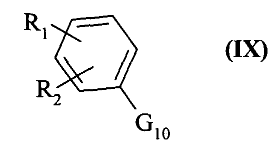 dans laquelle R₁ et R₂ ont les mêmes significations que dans la formule (I) et G₁₀ représentent un atome d'halogène choisi parmi chlore et brome ou un groupement triflate,
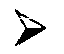 soit en présence de triphénylphosphine arsenic et d'un catalyseur au palladium, dans le cas où G₁₀ représente un groupement triflate,
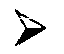 soit en présence d'un composé halogénocuivrique tel que CuBr₂ et d'un catalyseur au palladium, dans des conditions de solvant polaire, dans le cas où G₁₀ représente un atome d'halogène,
pour conduire également aux composés de formule (XXa) telle que décrite précédemment ;
• soit traités par du bis(pinacolato)di-borane, suivi d'une réaction d'oxydation, pour conduire aux composés de formule (XXII) : 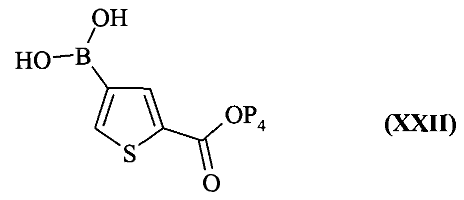 dans laquelle P₄ est tel que défini précédemment,
composés de formule (XXII) qui sont mis à réagir dans des conditions basiques de couplage au palladium avec un composé de formule (IX) tel que décrit précédemment pour conduire également aux composés de formule (XXa), tels que définis précédemment ;
• soit mis à réagir avec un composé de formule (IXa), (lesdits composés de formule (IXa) étant obtenus par traitement des composés de formule (IX) tels que définis précédemment, avec de l'hexaméthyldiétain en présence d'un catalyseur au palladium) : 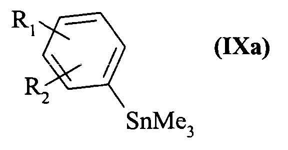 dans laquelle R₁, et R₂ sont tels que définis dans la formule (I),
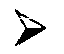 soit en présence de triphénylphosphine arsenic et d'un catalyseur au palladium, dans le cas où P₁ dans les composés de formule (XX) représente un groupement triflate,
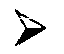 soit en présence d'un composé halogénocuivrique tel que CuBr₂ et d'un catalyseur au palladium, dans des conditions de solvant polaire, dans le cas où P₁ dans les composés de formule (XX) représente un atome d'halogène,
pour conduire également aux composés de formule (XXa) tels que définis précédemment ;
composés de formule (XXa) qui sont saponifiés dans des conditions d'hydrolyse basique, pour conduire aux composés de formule (XXb) : 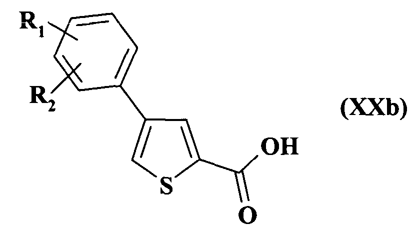 dans laquelle R₁ et R₂ sont tels que définis dans la formule (I),
composés de formule (XXb) qui sont soit transformés préalablement en chlorure d'acide correspondant par action du chlorure d'oxalyle, soit traités directement, dans des conditions de couplage peptidique en présence par exemple d'en agent de couplage et dans un milieu basique, avec un composé de formule (V) : 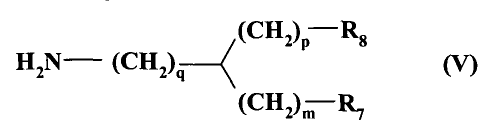 dans laquelle R₇, R₈, m, p et q ont les mêmes significations que dans les composés de formule (I),
pour conduire aux composés de formule (I): 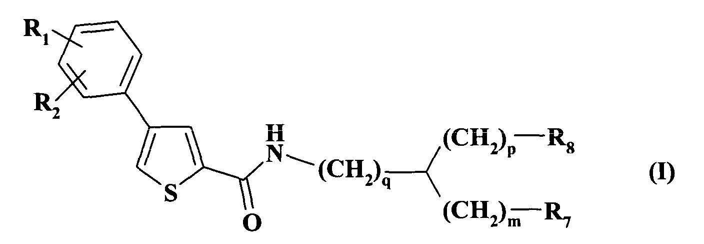 composés de formule (I), qui sont purifiés, le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

14. Compositions pharmaceutiques contenant comme principe actif au moins un composé de formule (I) selon l'une quelconque des revendications 1 à 10, seul ou en combinaison avec un ou plusieurs excipients ou véhicules inertes, non toxiques, pharmaceutiquement acceptables

15. Compositions pharmaceutiques selon la revendication 14 utiles pour la prévention ou le traitement des pathologies nécessitant l'action d'un inhibiteur de métalloprotéinase-12 et/ou de métalloprotéinase-13.

16. Compositions pharmaceutiques selon la revendication 14 utiles pour la prévention ou le traitement des pathologies nécessitant l'action d'un inhibiteur de métalloprotéinase-12.

17. Compositions pharmaceutiques selon la revendication 14 utiles pour la prévention ou le traitement des pathologies respiratoires choisies parmi les maladies pulmonaires obstructives chroniques (BPCO), l'emphysème, les bronchites chroniques, les inflammations pulmonaires chroniques, l'asthme, la mucoviscidose, la détresse respiratoire aigüe (ARDS), les allergies respiratoires dont la rhinite allergique, ainsi que les maladies liées à la production de TNFα incluant les maladies pulmonaires fibrotiques sévères, la sarcoïdose pulmonaire, et la silicose.

18. Compositions pharmaceutiques selon la revendication 14 utiles pour la prévention ou le traitement des pathologies liées à une inhibition de la métalloprotéinase-13 choisies parmi le cancer, l'ostéoporose, l'ostéoarthrite, l'arthrite, l'arthrite rhumatoïde, l'athéorosclérose, la sclérose en plaques, l'insuffisance cardiaque.

19. Compositions pharmaceutiques selon la revendication 14 utiles pour la prévention ou le traitement des maladies pulmonaires obstructives chroniques, de l'emphysème et des bronchites chroniques.

20. Compositions pharmaceutiques selon la revendication 14 utiles pour la prévention ou le traitement de l'emphysème lié au tabagisme.

21. Compositions pharmaceutiques selon la revendication 14 utiles pour la prévention ou le traitement de l'asthme.
